# EUROPÄISCHE PATENTSCHRIFT

(11) **EP 1 296 932 B1**
(45) Veröffentlichungstag und Bekanntmachung des Hinweises auf die Patenterteilung: **07.05.2008**
(21) Anmeldenummer: 01953178.9
(22) Anmeldetag: 26.06.2001
(51) Int. Cl.: C07C 211/62, C12N 15/10

(54) **VERWENDUNG VON KOMPOSITIONEN AUS KATIONISCHEN VERBINDUNGEN UND PROTONENDONOREN ZUR STABILISIERUNG UND/ODER ISOLIERUNG VON NUKLEINSÄUREN IN BZW. AUS MIKROORGANISMEN - WIE PROKARYONTEN, PILZEN, PROTOZOEN ODER ALGEN**
USE OF COMPOSITIONS CONSISTING OF CATIONIC COMPOUNDS AND PROTON DONORS FOR STABILISING AND/OR ISOLATING NUCLEIC ACIDS IN OR FROM MICRO-ORGANISMS SUCH AS PROKARYOTS, FUNGI, PROTOZOA OR ALGAE
UTILISATION DE COMPOSITIONS DE COMPOSES CATIONIQUES ET DE DONNEURS DE PROTONS POUR STABILISER DES ACIDES NUCLEIQUES DANS DES MICRO-ORGANISMES - COMME DES PROCARYOTES, DES CHAMPIGNONS, DES PROTOZOAIRES OU DES ALGUES - OU LES ISOLER DE CES MICRO-ORGANISMES

(30) Priorität: 27.06.2000 DE 10031236
(43) Veröffentlichungstag der Anmeldung: 02.04.2003
(73) Patentinhaber: QIAGEN GmbH, 40724 Hilden (DE)
(72) Erfinder: OELMÜLLER, Uwe, 40699 Erkrath (DE); WILLE, Tanja, 40723 Hilden (DE)
(86) Internationale Anmeldenummer: PCT/EP2001/007281
(87) Internationale Veröffentlichungsnummer: WO 2002/000600

(56) Entgegenhaltungen:
- EP-A- 0 606 712
- GB-A- 1 289 426
- US-A- 5 010 183
- US-A- 5 275 708
- US-A- 5 300 635
- US-A- 5 891 921
- REX CHISOLM: "Molecular Biology" INTERNET ARTICLE, [Online] 26. Januar 1995 (1995-01-26), XP002159472 Gefunden im Internet: <URL:<URL:http://www.dicty.cmb.nwu.edu/Chi s_I...%20Manual/molecular_biolog.htm > [gefunden am 2001-02-02]

## Beschreibung

Die vorliegende Erfindung betrifft eine neue Verwendung von Kompositionen die als einen wesentlichen Bestandteil eine kationische Verbindung der allgemeinen Formel

Y⁺R₁R₂R₃R₄ X⁻

worin
Y Stickstoff oder Phosphor
R₁, R₂, R₃ und R₄ unabhängig voneinander einen unverzweigten oder verzweigten C₁-C₂₀-Alkylrest und/oder einen C₆-C₂₀-Arylrest sowie einen C₇-C₂₆-Aralkylrest und
X⁻ ein Anion einer anorganischen oder organischen, ein- oder mehrbasischen Säure
bedeuten können
und mindestens einem Protonendonor als Additiv zur Stabilisierung und/oder Isolierung von RNA und/oder DNA aus Mikroorganismen - wie Prokaryonten, Pilzen, Protozoen oder Algen.

Bevorzugt sind Kompositionen, in denen die kationische Verbindungen aus einem Ammoniumsalz besteht, in dem R₁ einen höheren Alkylrest - vorzugsweise mit 12, 14 oder 16 - Kohlenstoffatomen und R₂, R₃ und R₄ jeweils eine Methylgruppe bedeutet.

Bevorzugt sind weiterhin Kompositionen, in denen R₁ eine Aralkylgruppe - vorzugsweise eine Benzylgruppe -, R₂ einen höheren Alkylrest - vorzugsweise mit 12, 14 oder 16 Kohlenstoffatomen - und R₃ und R₄ eine Methylgruppe bedeutet.

Als Anionen werden Bromid, Chlorid, Phosphat, Sulfat, Formiat, Acetat, Propionat, Oxalat, Malonat oder Succinat bevorzugt.

C₁-C₆-Alkyl steht im allgemeinen für einen verzweigten oder unverzweigten Kohlenwasserstoffrest mit 1 bis 6 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:

Methyl, Ethyl, Propyl, 1-Methylethyl (iso-Propyl), Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2methyl-propyl.

Höherer Alkylrest steht für einen verzweigten oder unverzweigten C₇-C₂₀-Alkylrest der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt: verzweigtes oder unverzweigtes Heptyl, Octyl, Nonyl, Decyl, Undecyl, Dodecyl, Tetradecyl, Hexadecyl, Dodecadecyl und Eicosyl.

C₃-C₆-Alkenyl steht im allgemeinen für einen verzweigten oder unverzweigten Kohlenwasserstoffrest mit 3 bis 6 Kohlenstoffatom(en), mit einer oder ggf. mehreren Doppelbindungen, der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:

2-Propenyl (Allyl), 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 3-Methyl-4-pentenyl, 4-Methyl-4-pentenyl, 1,1-Dimethyl-2-butenyl, 1,1,-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-Butenyl, 2,3-Dimethyl-3-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl.

C₃-C₆-Alkinyl steht im allgemeinen für einen verzweigten oder unverzweigten Kohlenwasserstoffrest mit 3 bis 6 Kohlenstoffatom(en), mit einer oder ggf. mehreren Dreifachbindungen, der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:

2-Propinyl (Propargyl), 2-Butinyl, 3-Butinyl, 1-Methyl-2-propinyl, 2-Methyl-2-propinyl, 2-Pentinyl, 3-pentinyl, 4-Pentinyl, 1-Methyl-2-butinyl, 2-Methyl-2-butinyl, 3-Methyl-2-butinyl, 1-Methyl-3-butinyl, 2-Methyl-3-butinyl, 3-Methyl-3-butinyl, 1,1-Dimethyl-2-propinyl, 1,2-Dimethyl-2-propinyl, 1-Ethyl-2-propinyl, 2-Hexinyl, 3-Hexinyl, 4-Hexinyl, 5-Hexinyl, 1-Methyl-2-pentinyl, 2-Methyl-2-pentinyl, 3-Methyl-2-pentinyl, 4-Methyl-2-pentinyl, 1-Methyl-3-pentinyl, 2-Methyl-3-pentinyl, 3-Methyl-3-pentinyl, 4-Methyl-3-pentinyl, 1-Methyl-4-pentinyl, 3-Methyl-4-pentinyl, 4-Methyl-4-pentinyl, 1,1-Dimethyl-2-butinyl, 1,1-Dimethyl-2-butinyl, 1,1-Dimethyl-3-butinyl, 1,2-Dimethyl-2-butinyl, 1,2-Dimethyl-3-butinyl, 1,3-Dimethyl-2-butinyl, 1,3-Dimethyl-3-butinyl, 2,2-Dimethyl-3-butinyl, 2,3-Dimethyl-2-butinyl, 2,3-Dimethyl-3-butinyl, 1-Ethyl-2-butinyl, 1-Ethyl-3-butinyl, 2-Ethyl-1-butinyl, 2-Ethyl-2-butinyl, 2-Ethyl-3-butinyl, 1,1,2-Trimethyl-2-propinyl, 1-Ethyl-1-methyl-2-propinyl und 1-Ethyl-2-methyl-2-propinyl.

Aryl steht - steht sofern nicht anders definiert - für einen aromatischen ein- oder mehrkemigen Rest mit 4 bis 22 C-Atomen, der ggf. ein oder zweit Heteroatome enthalten kann. Als Beispiele seien genannt: Phenyl, Naphthyl, Anthracyl bzw. Pyrol, Furan, Thiophen, Pyridin, Pyridazin, Pyrimidin oder Pyrazin, und der ggf. durch Halogen (F, Cl, Br, J) - vorzugsweise Fluor- oder durch eine Alkylgruppe unabhängig voneinander ein- oder mehrfach substituiert sein kann.

Aralkyl bedeutet einen ein oder mehrkemigen Arylrest im Sinne der vorstehenden Definition der über eine C₁-C₆-Alkylen-, C₃-C₆-Alkenylen- oder eine C₃-C₆-Alkinylenbrücke, für welche die Definition der C₁-C₆-Alkyl-, C₃-C₆-Akenyl und C₃-C₆-Alkinylgruppen entsprechend gelten, an die kationische Partialstruktur gebunden ist. Im Sinne der vorliegenden Erfindung wird die Benzylgruppe bevorzugt.

Als Gegenionen X- eignen sich bevorzugt alle Anionen von Halogenwasserstoffsäuren oder Anionen ein- oder zweibasischer organischer Säuren wie Acetat oder Oxalat, Malonat, Succinat oder Citrat.

Als Protonendonoren im Sinne der vorliegenden Erfindung sind in erster Linie gesättigte aliphatische Monocarbonsäuren, ungesättigte Alkenyl-carbonsäuren, gesättigte und/oder ungesättigte aliphatische C₂-C₆-Dicarbonsäuren, aliphatische Ketocarbonsäuren oder Ketodicarbonsäuren sowie Aminosäuren neben Mineralsäuren oder deren Salze allein oder in Kombination geeignet. Dabei können alle genannten organischen Säuren in unsubstituierter Form oder als substituierte Derivate eingesetzt werden, worunter - sofern nicht anders angegeben - die unsubstituierten oder ein bzw. mehrfach durch Hydroxyl-Gruppen substituierten Derivate bevorzugt werden.

Als gesättigte aliphatische Monocarbonsäuren im Sinne der vorliegenden Erfindung werden neben Ameisensäure vorzugsweise C₁-C₆-Alkyl-carbonsäuren verstanden, worunter Essigsäure, Propionsäure, n-Buttersäure, n-Valeriansäure, Isovaleriansäure, Ethyl-methyl-essigsäure (2-Methyl-buttersäure), 2,2-Dimethylpropionsäure (Pivalinsäure), n-Hexansäure, n-Octansäure, n-Decansäure sowie n-Dodecansäure (Laurinsäure) bevorzugt werden. Daneben können auch die sich von den genannten Säuren sich ableitenden Ketocarbonsäuren Verwendung finden.

Als ungesättigte Alkenyl-carbonsäuren im Sinne der Erfindung seien beispielsweise Acrylsäure (Propensäure), Methacrylsäure, Crotonsäure, iso-Crotonsäure sowie Vinylessigsäure genannt.

Bevorzugt im Sinne der vorliegenden Erfindung sind gesättigte aliphatische C₂-C₆-Dicarbonsäuren, wie zum Beispiel Oxalsäure, Malonsäure, Bersteinsäure, Glutarsäure oder Adipinsäure, worunter Oxalsäure und Bersteinsäure ganz besonders bevorzugt werden.

Besonders bevorzugt werden zur Lösung der erfidungsgemäßen Aufgabe aliphatische Hydroxi-di- und -tricarbonsäuren eingesetzt, worunter Tartronsäure, D-(+)-, L-(-)- oder DL-Äpfelsäure, (2R, 3R)-(+)-Weinsäure, (2S, 3S)-(-)-Weinsäure, meso-Weinsäure und Citronensäure ganz besonders bevorzugt werden.

Zur Lösung der vorliegenden Aufgabe eigenen sich daneben auch ungesättigte Dicarbonsäuren wie Malein- oder Fumarsäure oder ungesättigte Tricarbonsäuren, wie zum Beispiel Aconitsäure.

Im Sinne der vorliegenden Erfindung können jedoch auch aliphatische Ketodicarbonsäuren als Additive eingesetzt werden, wie z. B. Mesoxalsäure und Oxalessigsäure, worunter Oxalessigsäure ganz besonders bevorzugt wird.

Des weiteren können im Sinne der vorliegenden Erfindung Aminosäuren eingesetzt werden, worunter a-Aminosäuren - wie z. B. Aminoessigsäure (Glycin), α-Aminopropionsäure (Alanin), α-Amino-*iso*-valeriansäure (Valin), α-Amino-isocapronsäure (Leucin) und α-Amino-β-methylvaleriansäure (Isoleucin) bevorzugt werden. Besonders bevorzugt findet dabei Glycin Verwendung.

Die genannten Protonendonoren können als Einzelsubstanzen bzw. in Form der reinen Stereoisomeren als auch in Mischungen eingesetzt werden.

Als weitere Additive können im Sinne der vorliegenden Erfindung ebenfalls Mineralsäuren und deren Salze eingesetzt werden. Bevorzugt kommen dabei deren Salze von Mineralsäuren - wie Phosphorsäure oder Schwefelsäure - mit Alkalimetallen oder deren Ammoniumsalze zur Anwendung. Besonders bevorzugt finden dabei Phosphorsäure und Ammoniumsutfat Verwendung.

**Tab. 1**

| Bezeichnung | Formel |
|---|---|
| Essigsäure | CH₃-COOH |
| Oxalsäure | HOOC-COOH |
| Malonsäure | HOOC-CH₂-COOH |
| Tartronsäure | HOOC-CHOH-COOH |
| Bernsteinsäure | HOOC-CH₂-CH₂-COOH |
| Äpfelsäure | HOOC-CHOH-CH₂-COOH |
| Weinsäure | HOOC-CHOH-CHOH-COOH |
| Glutarsäure | HOOC-CH₂-CH₂-CH₂-COOH |
| Adipinsäure | HOOC-CH₂-CH₂-CH₂-CH₂-COOH |
| Zitronensäure | HOOC-CH₂-COHCOOH-CH₂-COOH |
| Maleinsäure | HOOC-CH=CH-COOH |
| Oxalessigsäure | HOOC-CO-CH₂-COOH |
| Glycin | H₂N-CH₂-COOH |

Das Additiv kann in unterschiedlichen Konzentrationen in der Komposition vorliegen. Des weiteren ist auch der Einsatz von Kombinationen verschiedener Additive möglich. - Dabei können in Abhängigkeit von der Natur des Additivs sich andere Konzentrationsbereiche als vorteilhaft erweisen . Daneben ist auch der Einsatz von Kombinationen verschiedener Additive möglich.

Die kationische Verbindung weist in der wässerigen Lösung der Komposition eine Konzentration in einem Bereich 0,01 % (w/v) und Sättigung, bevorzugt zwischen 0,1 % und 10 % (w/v) und Sättigung und besonders bevorzugt zwischen 0,5 und 8 % (w/v) und ganz besonders bevorzugt zwischen 2 und 6 % (w/v) auf.

Derartige Kompositionen werden in der Beschreibung der Deutschen Offenlegungsschrift 100 31 236, die der vorliegenden Patentanmeldung als Prioritätsanmeldung zugrundeliegt, sowie in den Patentansprüchen 1 bis 17 offenbart.

Naturgemäß werden bei der Zugabe einer Lösung von kationischer Verbindungen und Additiv die jeweiligen optimalen Konzentrationen durch das jeweilige Volumen der biologischen Probe und das Volumenverhältnis durch das Volumen der Stabilisierungslösung und das der biologischen Probe bestimmt.

Als Nukleinsäuren werden im Sinn der vorliegenden Erfindung - sofern nicht anders angegeben - Nukleinsäuren im breiteren Sinne verstanden, so z.B. Ribonukleinsäuren (RNA) die auch Desoxyribonukleinsäuren in allen Längen oder Konfigurationen - wie Doppelstrang, Einzelstrang, circuläre und lineare DNA umfassen sowie alle möglichen Unterarten, wie z.B. monomere Nukleotide, Oligomere, Plasmide, bakterielle DNA und RNA in prozessierter und unprozessierter Form.

Als biologische Probe können Lebensmittelproben oder Umweltproben, die freie oder gebundene Nukleinsäuren oder Nukleinsäure-haltige Mikroorganismen im : Sinne der Erfindung enthalten - wie z. B. Organismen (Ein- oder Mehrzeller; Insekten), Pflanzen und Pflanzenteile, Bakterien, Viren, Hefen und andere Pilze oder Prokaryonten - eingesetzt werden.

Des weiteren können als biologische Probe, welche die Mikroorganismen im Sinne der vorliegenden erfindung enthält, Plasma, Körperflüssigkeiten, wie beispielsweise Blut, Serum, Zellen, Leukozytenfraktionen, Crusta Phlogistica, Sputum, Urin, Sperma, Faeces, Abstriche, Punktate, Gewebeproben jeder Art - wie z. B. Biopsien -, Gewebeteile und Organe, Lebensmittelproben, die freie oder gebundene Nukleinsäuren oder Nukleinsäure-haltige Zellen enthalten, als Ausgangsmaterial dienen.

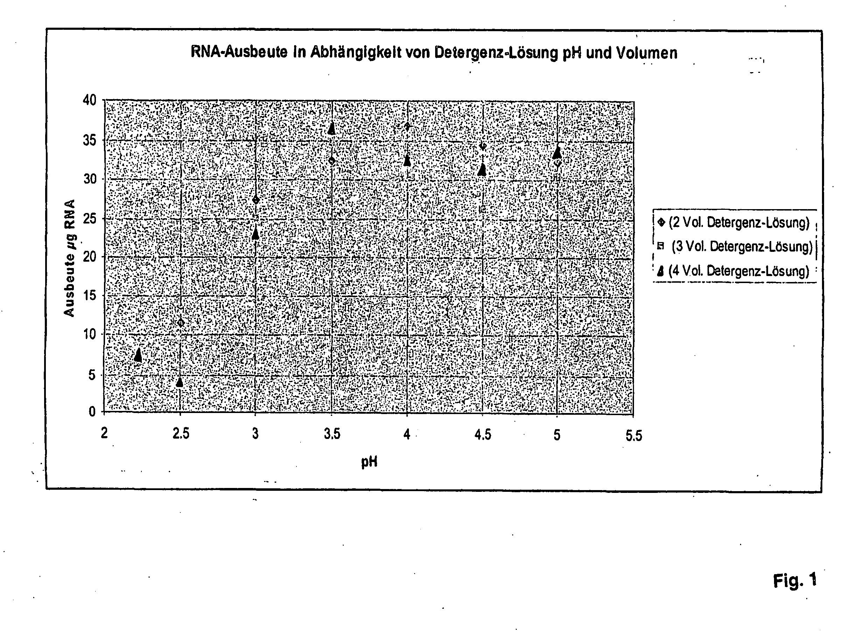

Abgesehen davon ist es möglich, Nukleinsäuren, die aus den oben genannten biologoischen Proben stammen und die eukaryontischer Herkunft sind, mit den erfindungsgemäßen Kompositionen zu stabilisieren. So ist es möglich, die in der Deutschen Patentanmeldung 100 31 236 (Seite 6, zweiter Absatz der ursprünglich eingereichten Unterlagen) genannten Materialen eukaryontischen Charakters gemäß der Lehre der vorliegenden Erfindung zu isolieren bzw. stabilisieren. Auf diese Weise läßt sich gemäß der Lehre der vorliegenden Erfindung Nukleinsäure eukaryontischer Herkunft, wie zum Beispiel aus Blut, Sputum oder Knochenmark - wie den Beispielen 1 bis 15 sowie den Figuren 1 bis 15 der Deutschen Patentanmeldung 100 31 236 offenbart, auf die hiermit inhaltlich Bezug genommen wird - zu entnehmen ist, erfolgreich stabilisieren.

Das Additiv kann in unterschiedlichen Konzentrationen in dem Stabilisierungsreagenz vorliegen; beispielsweise kann es in Mischungen der Stabilisierungslösung mit Blut in einem Volumenverhältnis von 1:1 - bevorzugt 3:1 in einer Konzentration von 50mM bis zur Sättigung , bevorzugt 100 bis 1 M und besonders bevorzugt in einer Konzentration von 200 - 500 mM zugegen sein. Dabei können in Abhängigkeit von der Natur des Additivs sich andere Konzentrationsbereiche als vorteilhaft erweisen . Daneben ist auch der Einsatz von Kombinationen verschiedener Additive möglich.

Die kationische Verbindung weist in der wässerigen Lösung der Komposition eine Konzentration in einem Bereich 0,01 Gew.% und Sättigung, bevorzugt zwischen 0,1 Gew.-% und Sättigung und besonders bevorzugt zwischen 0,5 Gew.% und 15 Gew.% und ganz besonders bevorzugt zwischen 2 Gew.-% und 10 Gew.-% auf.

Naturgemäß werden bei der Zugabe einer Lösung von kationischer Verbindungen und Additiv die jeweiligen optimalen Konzentrationen durch das Volumen der biologischen Probe und das Volumenverhältnis von Stabilisierungslösung zur biologischen Probe bestimmt.

Der pH-Wert der Mischung aus kationischer Verbindung und Additiv - vor dem Mischen mit der Probe - kann in Abhängigkeit von der Probe im allgemeinen über einen weiten pH Bereich (pH 2 bis 12) variiert werden und liegt bevorzugt in einem Intervall von pH 2 bis pH 10 und besonders bevorzugt in einem Intervall von pH 3 bis 8. Dabei ist der bevorzugte pH-Bereich abhängig von der eingesetzten biologischen Probe. - Für Blut, Plasma und Serum ist ein pH-Wert in einem Bereich zwischen pH 2 und pH 6 und besonders zwischen pH 3 und pH 4 bevorzugt.

Der pH-Wert der Mischung aus kationischer Verbindung und Additiv kann in Abhängigkeit von der Probe die Stabilisierung und/oder Isolierung von Nukleinsäuren in bzw. aus Mikroorganismen - wie Prokaryonten, Pilzen, Protozoen oder Algen - vorgesehen ist, im allgemeinen über einen weiten pH Bereich (pH 2 bis 12) variiert werden und liegt bevorzugt in einem Intervall von pH 2 bis pH 8 und besonders bevorzugt in einem Intervall von pH 2 bis 5. Dabei ist der bevorzugte pH-Bereich abhängig von der eingesetzten Probe.

Für biologische Proben, wie andere zelluläre Körperflüssigkeiten außer Blut, Plasma und Serum, oder z. B. Bakterien, Punktate, Zellen, Gewebe und weiterer biologischer Proben - wie oben beschrieben - liegt der pH-Wert in der Stabilisierungslösung bestehend aus kationischer Verbindung und Additiv bevorzugt in einem Bereich von pH 3 bis pH 10 und besonders bevorzugt in einem Intervall von pH 4 bis pH 8. Dabei ist bei allen pH-Angaben der pH-Wert vor dem Mischen mit der biologischen Probe zu verstehen.

Zur Stabilisierung von Nukleinsäuren in biologischen Proben kann die Probe mit einer Lösung, welche die kationische(n) Verbindung(en) und Additive enthält, vermischt werden. Dabei ist ein Zugabevolumen von 0,1 bis 10.000 Volumen der biologischen Probe möglich; bevorzugt wird ein Zugabevolumen in einem Bereich von 1 bis 1000 und ganz besonders bevorzugt in einem Intervall von 1 bis 100 Volumen. In Abhängigkeit von der Art der Probe - wie beispielsweise Proben aus feinen Nadelbiopsien oder Niedrigzellkulturen können jedoch u.U. auch wesentlich höhere Volumina in Frage kommen

Ebenso können die oben genannten kationischen Verbindungen und Additive auch als Feststoff zugesetzt werden, wenn die biologische Probe selbst Flüssigkeit zur Lösung des Feststoffes enthält (wie z. B. zellhaltige Körperflüssigkeiten, Zellen in Medium, Urin) oder Flüssigkeit, z. B. Wasser, zur Lösung des Feststoffes hinzu gegeben wird. Die Zugabe als Feststoff bietet den Vorteil, daß Feststoffe meist chemisch stabiler sind und ihre Zugabe zur Probe oft einfacher durchführbar ist.

Darüber hinaus ist insbesondere bei sehr kompakten biologischen Proben, wie beispielsweise Geweben, eine Zerkleinerung bzw. Homogenisation der Probe in der Stabilisierungslösung bzw. vor Mischung mit der Stabilisierungslösung möglich, um durch z. B. mechanische, chemische, physikalische oder enzymatische Einwirkung auf die Probe die Freisetzung der Nukleinsäuren oder einzelner Zellen bzw. Zellverbände durch Zerstörung einer kompakten Probe zu unterstützen. Eine mechanische Einwirkung kann z. B. mit einem elektrischen Messer, einer Kugelmühle oder durch Pressen durch eine Spritze geschehen, während sich geeignete Enzyme zur Einwirkung auf die Probe beispielsweise Hydrolasen, Proteasen oder Lipasen anbieten.

Daneben kann die Probe auf rein physikalischem Wege - beispielsweise mittels Ultraschall - vorbehandelt werden.

Die Vorbehandlung kann des weiteren auf chemischen Wege - entweder allein oder In Kombination mit rein physikalischen Methoden - erfolgen. Als Mittel zur Unterstützung der Lyse können z. B. aliphatische Alkohole - insbesondere Isopropanol - oder Aldehyde bzw. Dialdehyde - wie z. B. Glyoxal - oder auch Phenole oder Phenolderivate - wie z. B. 2-Biphenylol oder ionische, zwitterionische und nicht-ionische Verbindungen, - wie z. B. Sulfhydryl - oder reduzierende Reagenzien - wie z. B. Dithiothreitol und β-Mercaptoethanol - oder Phosphorsäurederivate - wie z. B. Tributylphosphat - oder aber chaotrope Reagenzien, wie z. B. Harnstoff, Guanidinium-thiocyanat oder Guanidiniumhydrochlorid - oder Salze einzeln oder in Kombination verwendet werden.

Weitere Möglichkeiten zur mechanischen, chemischen, physikalischen oder enzymatischen Einwirkung auf Proben sind dem Fachmann bekannt und sollen hier umfaßt sein.

Die Lagerung des Probenmaterials kann - den jeweiligen Bedürfnissen folgend - über längere Zeiträume, wie z. B. von 1 bis zu 14 Tage oder länger, bei Raumtemperatur aber auch bei erhöhten Temperaturen, wie z. B. 40°C oder mehr, und auch bei emiedrigten Temperaturen wie z. B. 4°C oder -20°C oder weniger erfolgen.

Im Anschluß an die Lagerung der biologischen Probe in der Lösung der o. g. Verbindungen können entweder direkt Nukleinsäure-Analysetechniken angeschlossen werden, oder es kann eine Aufreinigung der Nukleinsäuren aus der Probe stattfinden.

### Zum technologischen Hintergrund der Erfindung:

Die Untersuchung von RNA-Expressionsmustem bei Mikroorganismen mittels molekularbiologischer Methoden wie z. B. quantitative RT-PCR, NASBA, bDNA-Technolgoie oder Biochips und Northern Blotting findet Anwendung in der Grundlagenforschung bei der Analyse von Genexpressionen bei Procaryonten, sowie bei Protozoen, Pilzen und Algen und erlangt zudem wachsende Bedeutung beispielsweise in der medizinischen Diagnostik bei der Identifizierung von mikrobiellen Krankheitserregern, in der pharmazeutischen Industrie bei der Entwicklung und Evaluierung von Arzneimitteln, in der Biotechnologie bei der Herstellung rekombinanter Proteine für Forschung und therapeutische Anwendungen, in der Ökologie und Populations-Biologie oder auch in der Lebensmittelanalytik beim Nachweis von Verseuchungen mit Mikroorganismen.

Dabei besteht die Schwierigkeit, daß die Organismen zur Isolierung der Nukleinsäuren aus ihrer natürlichen Umgebung zur Gewinnung der zu untersuchenden Zellen entfernt werden und zum Ort der Isolierung der Nukleinsäuren transportiert werden müssen. Dabei besteht die große Gefahr, daß sich die RNA-Profile, aber auch die DNA verändern. Dies würde zu falschen Diagnosen bzw. Analysen z. B. von Genexpressionen in Bakterienkulturen oder z. B. auch in der medizinischen/klinischen Diagnostik bei der auf der Analyse von Nukleinsäuren basierenden Untersuchung von infiziertem Patientenmaterial (z. B. Proben aus Entzündungsherden) oder auch von mit Bakterien, Pilzen, Protozoen oder Algen verseuchten Lebensmitteln führen. In Lebensmittelproben oder klinischen Proben aus Patienten können die Mikroorganismen sogar absterben und somit die Nukleinsäuren, besonders die RNA vollständig abgebaut werden. Daher ist es von größter Bedeutung, daß die Nukleinsäuren, besonders die RNA unmittelbar bei der Entnahme der Probe stabilisiert werden.

Eine Besonderheit der Bakterien liegt in der extrem schnellen Anpassung ihrer Genexpression an die Milieubedingungen. Die daraus resultierenden kurzfristigen Änderungen der Genexpressionsmuster sind möglich aufgrund sehr kurzer Halbwertszeiten der zellulären mRNAs in Bakterien sowie deren Fähigkeit innerhalb weniger Sekunden oder Minuten neue RNA-Transkripte zu synthetisieren. Diese Anpassungsmechanismen stellen bei der Analyse prokaryontischer RNA-Expressionsmuster eine Schwierigkeit dar, da bereits während der Ernte der Zellen und der Prozedur der RNA-Präparation eine Veränderung der RNA-Expressiorismuster auftreten kann. Somit würde in nachfolgenden -Analysen nicht mehr das Expressionsmuster unter den definierten experimentellen .
Kulturbedingungen betrachtet, sondern ein RNA-Expressionsmuster, welches die Bedingungen während der Ernte, der Lyse oder der nachfolgenden Aufarbeitung der Zell-Lysate widerspiegelt.

Daneben läßt sich die Lehre der vorliegenden Erfindung auch auf andere RNA-Species als mRNA, wie z. B. rRNA, snRNA, tRNA, Low Molecular Weight (LMW) RNA-Species aber auch auf DNA, wie z. B. genomische DNA (gDNA) anwenden.

Klassische Methoden zur RNA-Isolierung aus Mikroorganismen - wie Prokaryonten, Pilzen, Protozoen oder Algen- basieren z. B. auf dem Einsatz organischer Lösungsmittel wie Phenol und Chloroform(Trichlormethan), auf Verwendung chaotroper Salze oder aus Kombinationen dieser Substanzen. Bei allen bisher bekannten Methoden zur Nukleinsäure-Isolierung aus Prokaryonten, Pilzen, Protozoen oder Algen müssen die Zellen zunächst durch Zentrifugation oder Filtration aus dem Kulturmedium angereichert werden, bevor die weitere Aufarbeitung erfolgen kann. Während dieses ersten Arbeitsschrittes kommt es in sehr zahlreichen Fällen in den Zellen aufgrund der veränderten Milieubedingungen (z.B. Temperaturänderung, mechanische Belastung durch Zentrifugation bzw. Filtration, veränderte Gasatmosphäre) zu einer Änderung des Genexpressionsmusters, so daß das Genexpressionsmuster der Zellen nicht die definierten Kulturbedingungen widerspiegelt, sondern die Bedingungen der Prozedur der Nukleinsäureisolierung reflektiert. Somit wird die Validität nachfolgender Analysen fraglich.

Für die Veränderung des Expressionsmusters sind in erster Linie der unspezifische Abbau der RNA durch RNasen oder durch chemische Einflüsse - wie Deprotonierung - oder der sequenzspezifische RNA-Abbäu - durch RNasen, die spezielle Sequenzen abbauen -, verantwortlich. Daneben hat die Neusynthese von RNA ebenfalls einen nachteiligen und deshalb unerwünschten Einfluß.

Häufig wird versucht, diesen Einfluß durch eine sehr schnelle Zellemte und einen schnellen Zellaufschluß zu minimieren, jedoch ist es auf diesem Weg nicht möglich, die Veränderung des Genexpressionsmusters komplett zu unterbinden. Weiterhin wird somit die gleichzeitige Aufarbeitung größerer Probenzahlen unmöglich. Zudem wurde ein enzymatischer Zellaufschluß häufig vermieden, weil dieser nur vor der Zugabe organischer Lösungsmittel oder konzentrierter chaotroper Salzlösungen möglich war, und mindestens 3 Minuten Zeit beansprucht. Somit wäre während eines enzymatischen Zellaufschlusses gleichzeitig eine enzymatische RNA-Degradierung sowie eine RNA-Neusynthese möglich, wodurch das Genexpressionsmuster der Zellen verändert worden wäre. Aus diesem Grund war es für nachfolgende Genexpressionsanalysen stets nachteilig, einen enzymatischen Zellaufschluß durchzuführen.

Eine weitere Schwierigkeit bei der Nukleinsäure-Isolierung (RNA und DNA) aus Prokaryonten, Pilzen, Algen und auch Protozoen besteht in der Lyse der Zellen, da hierzu die Zellwand aufgeschlossen werden muß. Eine verbreitete Methode besteht z. B. im Verdau des Mureins in der prokaryontischen Zellwand mit dem Enzym

Lysozym; alternativ können jedoch auch andere Enzyme eingesetzt werden wie z.B. Lysostaphin oder Proteinasen. Während des Verdaus müssen in der zu bearbeitenden Probe Bedingungen vorliegen, die die enzymatische Aktivität des entsprechenden Enzyms gewährleisten. Gleichzeitig erlauben solche Bedingungen meist aber auch eine Spaltung der bakteriellen mRNA durch RNasen oder die chemische Hydrolyse der Nukleinsäuren, so daß häufig degradierte Nukleinsäuren aus den entsprechenden Präparationen resultieren. Weiterhin kann nicht ausgeschlossen werden, daß während dieses enzymatischen Aufschlusses der Zellen die Synthese von Nukleinsäuren stattfindet, wodurch zusätzlich das RNA-Expressionsmuster verändert würde.

Zur Aufgabe der vorliegenden Erfindung:

Die allgemeine Aufgabe der vorliegenden Erfindung besteht darin, die geschilderten und aus dem Stand der Technik bekannten Nachteile zu vermeiden.

Der vorliegenden Erfindung liegt demgemäß die Aufgabe zugrunde, bei der Nukleinsäure-Isolierung aus Mikroorganismen wie Bakterien, Pilzen - wie z.B. Hefen -, Protozoen oder Algen eine aufarbeitungsbedingte Änderung des Genexpressionsmusters zu vermeiden, so daß das Genexpressionsmuster der Zellen die definierten Kulturbedingungen oder die Bedingungen in der ursprünglichen Probe, wie z. B. einer Patienten- oder Lebensmittelprobe, widerspiegelt, nicht die durch die Bedingungen der Zellemte bzw. der Prozedur der Nukleinsäureisolierung hervorgerufenen Veränderungen, um somit die Validität der ggf. nachfolgenden Analysen zu sichern.

Eine weitere Aufgabe der vorliegenden Erfindung besteht darin, die zeitlich parallele Aufarbeitung größerer Probenzahlen zur Isolierung von RNA und DNA zu ermöglichen.

Der vorliegenden Erfindung liegt daneben die Aufgabe zugrunde, eine Komposition in Form einer Stabilisierungslösung bereit zu stellen, deren Bestandteile nicht gesundheitsschädlich sind und damit z. B. auch für eine Stabilisierung von RNA und/oder DNA in biologischem Probenmaterial während des Transportes vom Ort der Entnahme zu einem Labor ohne Gesundheitsgefahren - wie sie z.B. bei der Verwendung von Phenol erwachsen - für das mit der Probenbearbeitung befaßte Personal eingesetzt werden kann.

Die Lehre der vorliegenden Erfindung ist unter den Prokayonten - neben den Archaebakterien (wie z.B. Methanothermobacter marbirgensis) - insbesondere auf die Eubakterien anwendbar. Unter die Eubakterien fallen Gram-positive wie auch Gram-negative Bakterien, wie auch Phototrope Bakterien bzw. Clamydien, Mycoplasma (wie z.B. Mycoplasma penetrans), Rikettsien, Spirillen und Spirochaeten (wie z. B. Borrelia burgdorferi), auf die sich die erfindungsgemäße Lehre anwenden läßt.

Unter den Gram-positiven Eubakterien sind insbesondere Bacillus (wie z.B. Bacillus subtilis), Staphylococcus (wie zum Beispiel Staphylococcus aureus oder Staphylococcus epidermis), Streptomyces (wie zum Beispiel Streptomyces coelicotor oder Streptomyces lividans), Flavobakterium (wie zum Beispiel Flavobakterium johnsoniae), Mycobakterium (wie zum Beispiel Mycobakterium avium) oder Streptococcus zu nennen.

Des weiteren ist die Lehre der vorliegenden Erfindung auf folgende Gram-positive Eubakterien anwendbar:
Clostridien (wie z. B. C. difficile, C. tetani und C. perfringens)
Lysteria
Peptococcus
Peptostreptococcus
Enterococcus
Corynebacterium (wie z. B. C. diphteriae oder C. glutamicum)
Propionibakterium
Lactobacillus

Unter den Gram-negativen Eubakterien sind insbesondere Escherichia (wie zum Beispiel Escherichia coli), Pseudomonas (wie zum Beispiel Pseudomonas aeroginosa, Pseudomonas putida oder Pseudomonas syringae), Klebsiella (wie zum Beispiel Klebsiella pneumoniae), Salmonella (wie zum Beispiel Salmonella typhimurium), Sinorhizobium (wie zum Beispiel Sinorhizobium meliloti) oder Campylobacter.

Daneben läßt sich die Lehre der vorliegenden Erfindung auf folgende Gram-negative Bakterien anwenden:
Neisseria (wie z. B. Neisseria gonorrhoae oder N. meningitidis)
Vibrio (wie z. B. Vibrio cholerae)
Shigella
Serratia
Enterobacter
Acinetobacter
Proteus
Yersinia
Brucella (wie z. B. B. abortus)
Haemophilus (wie z. B. H. influenza)
Bacteroides
Campylobacter
Helicobacter (wie z. B. H. pylori)
Bordetella
Legionella
Pasteurella

Unter den Eukaryonten sind insbesondere zu nennen Pilze, darunter die Pilzgruppe der Dermatophyten, der Hefen, der Schimmelpilze und der biphasischen Pilze.

Unter den Hefen sind insbesondere zu nennen die Gattungen Saccaromyces (wie zum Beispiel Saccaromyces cerevisiae), Candida (wie z. B. Candida albicans), Cryptococus (wie z. B. Cryptococcus neoformans). Unter den Schimmelpilzen sind insbesondere zu nennen die Gattungen Aspergillus (wie zum Beispiel Aspergillus fumigatus) oder Penicillium oder Mucor.

Ferner sind als Beispiele für Eucaryonten Algen und Protozoen - wie z. B. Trypanosomen, Toxoplasmen, Amoeben, Plasmodien, Flagellaten - zu nennen, auf die sich die Lehre der Erfindung anwendbar ist.

Zur erfindungsgemäßen Lösung der Aufgabe:

Die vorgenannten Aufgaben der vorliegenden Erfindung werden dadurch gelöst, daß man eine definierte Bakterien- Pilz- , Protozoen- oder Algenkultur oder eine Probe, die Bakterien und/oder Pilze und/oder Protozoen und/oder Algen enthält mit der Komposition - bzw. mit ihrer wässerigen Lösung - umfassend eine kationische Verbindung der allgemeinen Formel 1 und mindestens einem Protonendonor - in Kontakt bringt.

Zur anschließenden Zellemte und weiteren Aufarbeitung der Probe ist ein enzymatischer Verdau der Zellwand, z. B. des Mureingrundgerüsts der bakteriellen Zellwand mit Lysozym möglich, wobei die Nukleinsäuren in der Probe keiner enzymatischen oder chemischen Degradierung unterliegen bzw. keine Neusynthese von Nukleinsäuren stattfindet, so daß eine Änderung des RNA-Expressionsmusters verhindert wird.

Alternativ sind auch andere enzymatische Zellaufschlußmethoden denkbar, beispielsweise unter Einsatz von Lysostaphin, Proteinase K, oder auch ein Detergenz-vermittelter Zellaufschluß bzw. Kombinationen der genannten Aufschlußmethoden, auch mit mechanischen Aufschlußmethoden.

Dabei bietet ein enzymatischer Zellaufschluß, im Gegensatz zu mechanischen Aufschlußmethoden - wie beispielsweise durch Einsatz einer Kugelmühle oder des Zermörserns in flüssigem Stickstoff -, prinzipiell den Vorteil, relativ gut automatisierbar zu sein. Des weiteren erlaubt ein enzymatischer Zellaufschluß einen hohen Probendurchsatz und minimiert im Vergleich zu den aus dem Stand der Technik ebenfalls bekannten mechanischen Zellaufschluß-Methoden die Gefahr von Kreuzkontaminationen.

Neben dem enzymatischen Zellaufschluß von Bakterien bietet sich daneben auch der Zellaufschluß von Hefe-Zellen mit Hilfe von Zymolase oder Lyticase, oder auch ein anderer Aufschluß eukaryontischer Zellen mit Proteinase oder anderen

Enzymen bzw. mit Hilfe von Detergenzien nach Stabilisierung der Zellen mit den erfindungsgemäßen Kompositionen an.

Obwohl aus den angeführten Gründen prinzipiell ein enzymatischer Zellaufschluß vorteilhaft ist, wird dieses Verfahren für Analysen von Genexpressionsmustern kaum angewendet, da während dieses Arbeitsschritts bei der Durchführung herkömmlicher Präparationsmethoden eine Änderung des RNA-Expressionsmusters gerechnet werden muß. Die Anwendung des hier beschriebenen Verfahrens bietet eine Möglichkeit zur Lösung dieses Problems, indem die RNA in den Zellen bereits vor der Zellemte stabilisiert wird. In einem nachfolgenden Arbeitsschritt ist ein enzymatischer Zellaufschluß möglich, wobei eine enzymatische oder chemische Degradierung der RNA sowie eine Neusynthese unterbunden wird.

Alternativ zu einem enzymatischen Zellaufschluß kann auch ein mechanischer, thermischer oder chemischer Zellaufschluß erfolgen, sowie Kombinationen aus einem oder mehreren der genannten Aufschlußmethoden.

Nach der Stabilisierung und dem Zellaufschluß können zur weiteren Aufarbeitung der Probe die aus dem Stand der Technik bekannten Verfahren zur Isolierung von Nukleinsäuren auf Basis modifizierter Silica-Materialien eingesetzt werden.

Durch die vorliegende Erfindung wird eine Möglichkeit zur weiteren Aufarbeitung der Probe - beispielsweise in einer RNA-Isolierung mit Hilfe organischer Lösungsmittel, chaotroper Salze oder durch Aussalzen der Nukleinsäuren oder durch Einsatz magnetischer Partikel oder über Hybrid-Capture-Verfahren-eröffnet:

Im Vergleich zu bisherigen aus dem Stand der Technik bekannten RNA-Extraktionsmethoden - wie TRIzol oder RNeasy - wird durch den Einsatz der erfindungsgemäßen Komposition aus einem kationischem Detergenz der allgemeinen Formel I und einem Protonendonor - in Form eines Additivs, bevorzugt in Form einer aliphatischen Carbonsäure, besonders bevorzugt einer Dicarbonsäure, worunter Weinsäure ganz besonders bevorzugt wird - eine Ausbeute erzielt, welche derjenigen der o.a. herkömmlichen Methoden beispielsweise um das 2- bis 3-fache übersteigt.

Diese hohe RNA-Ausbeute ist insbesondere vorteilhaft bei der Analyse niedrig exprimierter RNA-Transkripte oder solcher RNA-Transkripte, die nur von einer Subgruppe einer zu analysierenden Bakterienpopulation exprimiert werden. Zudem gibt es bisher keine praktikable Methode zur mRNA-Isolierung aus Bakterien, so daß man bei der Analyse bakterieller mRNAs mit einem starken Hintergrund anderer RNA-Species (rRNA, tRNA, snRNPs) arbeitet. Bei derartigen Fragestellungen ist von einer Steigerung der Sensitivität der analytischen Verfahren aufgrund der gesteigerten Ausbeute auszugehen.

Die Vorteile der Erfindung liegen insbesondere bei allen Anwendungen, in denen Analysen von Genexpressionsmustern in Mikroorganismen (Procaryonten, Protozoen, Pilzen, Algen) durchgeführt werden sollen. Dieses umfaßt beispielsweise wissenschaftliche Fragestellungen, die zum basalen Verständnis der Regulation der prokaryontischer Genexpression beitragen, als auch Studien, in denen beispielsweise die Beziehung zwischen der Expression ausgewählter Gene und der Pathogenität von Bakterien analysiert wird. Letzterer Fragestellung ist insbesondere in der Diagnostik und zur Behandlung bakterieller Infektionen relevant.

Ein weiteres wichtiges Anwendungsgebiet der Erfindung liegt in der Genexpressionsanalyse von Prokaryonten, Protozoen und Pilzen in der pharmazeutischen Forschung und Entwicklung. Die Stabilisierung z. B. prokaryontischer RNA-Expressionsmuster vereinfacht erheblich die Analyse von Transkriptspiegeln oder kompletter Expressionsmuster etwa im Rahmen von Experimenten, in denen die Zeitabhängigkeit der Genexpression dargestellt werden soll. Weiterhin wird die Identifizierung und Quantifizierung von Spezies in komplexen Populationen, beispielsweise von Bakterien in einer Bodenprobe oder von Krankheitserregern in Proben von Patienten erheblich erleichtert. Darüber hinaus erstreckt sich das Anwendungspotential auch auf weitere analytische Bereiche wie z.B. auf die Lebensmittelanalytik.

Durch die Stabilisierung von Nukleinsäuren mit Hilfe der erfindungsgemäßen Komposition aus einer oder mehreren kationischen Verbindung(en) und einem oder mehreren Additiv(en) wird erreicht, daß die Nukleinsäuren in einer Probe auch bei längerer Lagerung oder während eines Transports sich nicht verändern. Somit wird die Genauigkeit später durchgeführter Tests deutlich erhöht. In bestimmten Fällen, wenn z. B. das Probenmaterial über weite Strecken transportiert oder länger gelagert werden muß - macht das erfindungsgemäße Verfahren diese Tests nach einem derartigen Zeitraum überhaupt erst möglich.

Die Vorteile dieser Erfindung liegen insbesondere sowohl im Bereich der Forschung, z. B. für die Analyse von Transkriptspiegeln, die direkt nach der Entnahme fixiert werden müssen, als auch im Bereich klinischer Analysen - wie z. B. molekulare Diagnostik -, wo Patientenproben nach der Entnahme während Lagerung und Transport bis zur Analyse ebenfalls stabilisiert werden müssen.

Daneben findet die Isolierung und Stabilisierung von Nukleinsäuren Anwendung in der Tumordiagnostik, in der Diagnostik erblich bedingter Krankheiten sowie in der Virusdiagnostik und dem Virus-Monitoring und der Diagnose und dem Monitoring anderer infektiöser Erreger, sowie in der Analyse von Genexpressionsmustem.

Erläuterung der Figuren:
Fig. 1 zeigt in graphischer Darstellung die Abhängigkeit der RNA-Ausbeute vom pH-Wert der Detergenz-Lösung und vom Volumenverhältnis zwischen Kultur und Detergenz-Lösung.
Fig. 2 zeigt in graphischer Darstellung die Abhängigkeit der RNA-Ausbeute der verschiedenen Protokoll-Varianten.
Fig. 3 zeigt in graphischer Darstellung die RNA-Ausbeute in Abhängigkeit vom Volumen der wässerigen Lösung der erfidnungsgemäßen Verbindung.
Fig. 4 zeigt das Ergebnis der denaturierenden Agarose-Gelelektrophorese und ompA Northern Blot Analyse von *E. coli* RNA isoliert mit Lösungen verschiedener Volumina von Lösungen der Komposition aus kationischer Verbindung und Protonendonor in verschiedenen Konzentrationen.
Fig. 5 zeigt die ompA ("outer membrane protein A") Northern Blot Analyse von *E. coli* RNA isoliert nach Rifampicin-Zugabe mit bzw. ohne Lösung der erfindungsgemäßen Komposition.
Fig. 6 zeigt die bla (β-lactamase) Northern Blot Analyse von *E. coli* RNA isoliert nach Rifampicin-Zugabe mit bzw. ohne Verwendung der Komposition aus kationischer Verbindung und Additiv bzw. deren wässeriger Lösung.

Die nachfolgenden Beispiele sollen die vorliegende Erfindung erläutern:

### Beispiel 1

### RNA-Isolierung aus E. coli

Eine wässerige Lösung bestehend aus 4% (w/v) Tetradecyltrimethylammoniumoxalat
200mM Weinsäure wird mit Natronlauge auf folgende pH-Werte eingestellt: 2,2 (ohne Zugabe von NaOH); 2,5; 3,0; 3,5; 4,0; 4,5 und 5,0. 25.
Zur Versuchsdurchführung werden pro Ansatz zu 400µl einer Kultur von *E. coli* in LB-Medium je 2; 3 bzw. 4 Volumen der Detergenz-Lösung der verschiedenen pH-Werte zupipettiert, und die RNA-Isolierung nach folgendem Protokoll durchgeführt:
- Zugabe von 400µl der *E. coli* Kultur zur vorgelegten Detergenz-Lösung, vortexen
- Zentrifugation 5000 x g 10min bei 4°C
- Überstand dekantieren
- Pellet in 1 ml H₂O resuspendieren
- Zentrifugation 5000 x g 10 min bei 4°C
- Überstand dekantieren
- Pellet in 100µl TE-Puffer¹⁾ mit 400µg/ml Lysozym resuspendieren
- Inkubation 5min bei Raumtemperatur
- Zugabe von 300µl RLT -Puffer²⁾ vortexen
- Zugabe von 260µl H₂O, vortexen
- Zugabe von 40µl Proteinase K (18mg/ml), vortexen
- Inkubation 10min 55°C
- Zentrifugation 3min 14000 xg
   ¹⁾TE Puffer besteht aus 10 mM Tris-HCl und 1 mM EDTA, der bei einem pH-Wert von 8 puffert.
   ²⁾Unter RLT-Puffer ist ein handelsüblicher Puffer (erhältlich von der Fa. QIAGEN, Hilden) auf der Basis eines Guanidiniumsalzes - wie z.B. Guanidiniumisothiocyanat - und eines Alkalisalzes einer mehrbasischen organischen Säure sowie β-Mercaptoethanol zu verstehen, der bei pH 7 puffert.
- Überstand abnehmen, Zugabe von 350µl 100% Ethanol, vortexen-Laden der Lösung auf RNeasy Mini spin column
- weitere Aufarbeitung wie aus dem Stand der Technik bekannt - z.B. wie im RNeasy® Mini Protokoll der Fa. QIAGEN, Hilden zur Isolierung von total RNA aus Bakterien ab Schritt 5 beschrieben.

Fig. 1 zeigt die RNA-Ausbeute in Abhängigkeit vom pH-Wert der Detergenz-Lösung und vom Volumenverhältnis zwischen Kultur und Detergens-Lösung

Die erhaltenen Resultate zeigen außerdem, daß sich die höchsten Ausbeuten an RNA dann erzielen lassen, wenn die wässerige Lösung der erfindungsgemäßen Komposition einen pH-Wert in einem Intervall von 3,5 bis 5,0 aufweist.

Die Intaktheit der RNA wird per Agarose-Gelelektrophorese analysiert. In allen Fällen werden intakte ribosomale RNA Banden gefunden.

### Beispiel 2

### RNA-Isolierung aus E. coli mit verschiedenen Protokollvarianten

Ausgangsmaterial für die unter diesem Beispiel zusammengefaßten Experimente ist wiederum eine in LB-Medium angezogene *E. coli* Kultur. Alle verschiedenen Protokoll-Varianten werden unter Einsatz von je 1,5×10⁸ sowie 3×10⁸ Zellen durchgeführt. Für diese Versuchs-Reihe wird eine wässerige Lösung der errfindungsgemäßen Komposition mit folgender Zusammensetzung eingesetzt:
4% (w/v) Tetradecyl-trimethyl-ammoniumoxalat
200mM Weinsäure
mit einem pH-wert von 4,0

Ausgehend von folgenden Partiallschritten des RNA-IsolierungsrProtokolls werden verschiedene Protokoll-Varianten erstellt:
1) Zellemte
   Zugabe von 3 Volumen Detergenz-Lösung zur *E. coli* Kultur, vortexen, Zentrifugation, 5000 × g 10 min bei 4°C, Überstand dekantieren
2) Waschen des Pellets
   Pellet in 1 ml H₂O resuspendieren, Zentrifugation 5000 × g 10min bei 4°C, Überstand dekantieren
3) Lysozym-Verdau
   Pellet in 100µl TE-Puffer mit 400µg/ml Lysozym resuspendieren, Inkubation 5min bei Raumtemperatur
4) Bindebedingungen einstellen
   Zugabe von 350µl RLT-Puffer, vortexen, Zugabe von 250µl 100% Ethanol
5) Proteinase K Verdau
   Zugabe von 300µl RLT-Puffer, vortexen, Zugabe von 260µl H2O, Zugabe von 40µl Proteinase K (18mg/ml), vortexen, Inkubation 10min 55°C, Zentrifugation 3min 14000 × g, Überstand abnehmen, Zugabe von 350µl 100% Ethanol, vortexen
6) Lysozym-Verdau und Proteinase K Verdau in verdünntem RLT-Puffer
   Zugabe von 300µl RLTPuffer, vortexen, Zugabe von 160pl H₂O, vortexen, Zugabe von 1 00µl TE-Puffer mit 400µg/ml Lysozym, Inkubation 5 min bei Raumtemperatur, Zugabe von 40µl Proteinase K (18mg/ml), vortexen, Inkubation 10 min 55°C, Zentrifugation 3 min 14000 xg, Überstand abnehmen, Zugabe von 350µl 100% Ethanol, vortexen
7) Aufarbeitung mittels einer ggf. modifizierten Silicasäule - wie z.B. mit der RNeasy Mini spin column (erhältlich von der Firma QIAGEN, Hilden)
   Laden der Lösung auf die Säule, weitere Aufarbeitung It. RNeasy Mini Protokoll zur Isolierung von total RNA aus Bakterien, Schritt 5

Das Ausgangsprotokoll wird mit folgenden Protokoll-Varianten verglichen:
Variante 1: Schritte 1); 3); 4) und 7)
Variante 2: Schritte 1); 2); 3); 4) und 7)
Variante 3: Schritte 1); 2), 3); 5) und 7) (Ausgangsprotokoll wie unter Beispiel 1)
Variante 4: Schritte 1); 6) und 7)

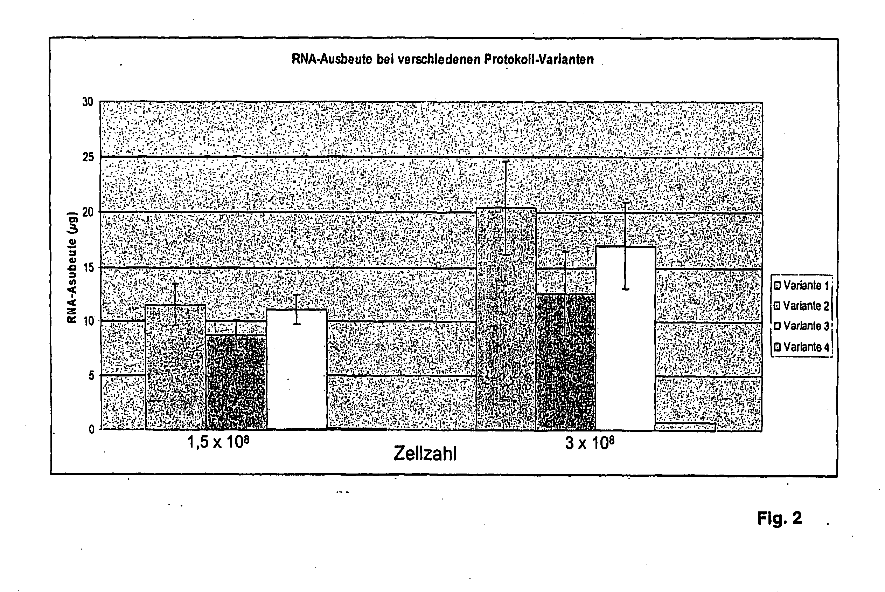

Fig. 2 zeigt die RNA-Ausbeute der verschiedenen Protokoll-Varianten

Die Ergebnisse dieser Experimente liefern einen deutlichen Hinweis dafür, daß die Durchführung der Protokoll-Variante 1 bezüglich der RNA-Ausbeute vergleichbar ist mit dem Ausgangsprotokoll (Variante 3). In dieser Protokoll-Variante wird auf einen Waschschritt sowie auf den Proteinase K Verdau verzichtet. Insgesamt wird somit die Aufarbeitungs-Prozedur wesentlich verkürzt, so daß diese Protokoll-Variante in folgenden Beispielen als Standardmethode eingesetzt wird.

### Beispiel 3

RNA-Isolierung aus E. coli mit verschiedenen Volumenverhältnissen von Kultur und wässerriger Lösung der erfindungsgmeäßen Komposition

Es werden wässerige Lösungen der erfindungsgemäßen Komposition mit folgender Zusammensetzungen verwendet:
- Lösung QCX 1: 4% (w/v) Tetradecyltrimethylammoniumoxalat 200mM Weinsäure pH 4,0
- Lösung QCX 2: 6% (w/v) Tetradecyltrimethylammoniumoxalat 300mM Weinsäure pH 4,0
- Lösung QCX 3: 8% (w/v) Tetradecyltrimethylammoniumoxalat 400mM Weinsäure pH 4,0
- Lösung QCX 4: 15% (w/v) Tetradecyltrimethylammoniumoxalat 750mM Weinsäure pH 4,0

Jeweils 400µl einer in LB-Medium (10g Trypton; 5g Hefeextrakt; 10g NaCl; H₂O ad 1000ml) gewachsenen *E. coli* Kultur werden mit 2; 3 bzw. 4 Volumina der jeweiligen Lösungen versetzt, und nach dem in Beispiel 2 definierten Standardprotokoll aufgearbeitet.

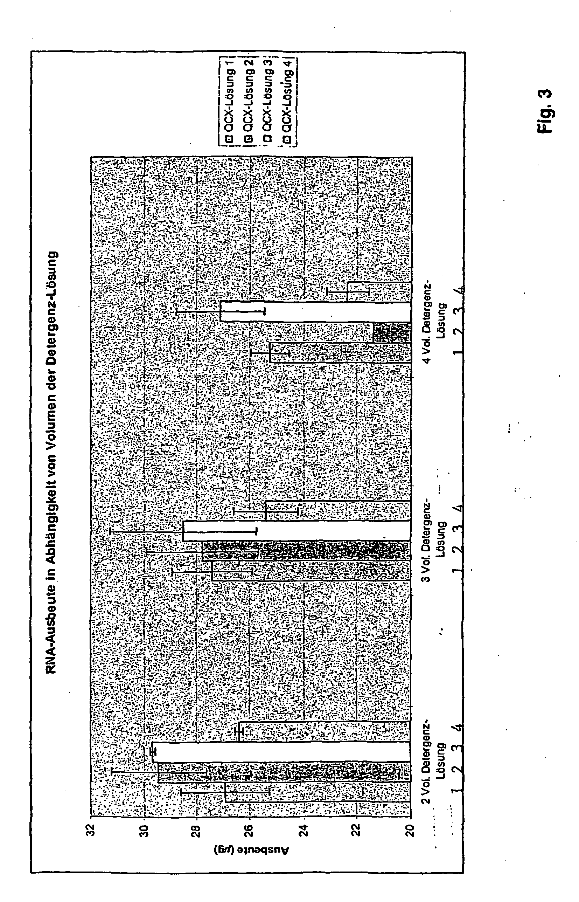

Fig. 3 zeigt die RNA-Ausbeute in Abhängigkeit vom Volumen der wässerigen Lösung der kationischen Verbindung gemäß Formel 1 und Additiv.

Wie aus den experimentellen Befunden hervorgeht, sind die RNA-Ausbeuten bei Einsatz von 2 bzw. 3 Volumina der verschiedenen Detergenz-Lösungen durchaus vergleichbar. Bei Einsatz von 4 Volumen der oben beschriebenen Lösungen kommt es zu einem gewissen Verlust an RNA-Ausbeute. Innerhalb der Ansätze unter Einsatz von 2 bzw. 3 Volumina der Detergenz-Lösung werden mit den Lösungen 1; 2 und 3 vergleichbar hohe Ausbeuten erzielt.

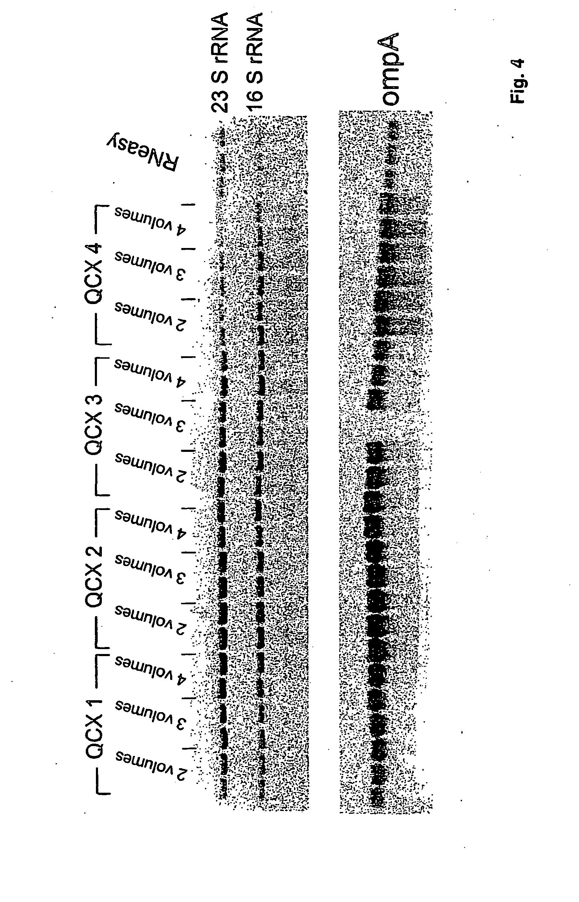

Um die Intaktheit der isolierten RNA beurteilen zu können, wird die RNA auf denaturierenden Agarosegelen aufgetrennt und anschließend eine Northern Blot Analyse durchgeführt (vgl. Fig. 4!).

Die visuelle Analyse der rRNA-Banden zeigt größtenteils intakte ribosomale RNA-Banden auf dem Agarosegel. Lediglich die bei Verwendung von Lösung 4 erhaltenen Banden deuten auf eine teilweise Degradierung der RNA. Dieser Befund wird durch eine Northern Blot Analyse gesichert, bei der mit einer gegen die ompA ("outer membrane protein A"*) mRNA aus *E*. *coli* gerichtete Sonde hybridisiert wird.

*Bei der ompA mRNA handelt es sich mit einer Halbwertszeit von 15 min um ein relativ langlebiges RNA-Transkript (Nature 1984, 312: 75 - 77).

Wieder zeigt sich eine Degradierung der RNA, die mit Hilfe der Lösung 4 isoliert wird. Bei Vergleich der übrigen RNA-Proben zeigen sich die schärfsten ompA mRNA-Banden in den Spuren, in denen RNA mit der Lösung 1 isoliert wird. Insgesamt weisen die RNA-Proben, die mit den Lösungen 1 - 3 isoliert worden sind, jedoch nur sehr geringe Unterschiede bezüglich der RNA Qualität auf.

Fig. 4 gibt das Ergebnis der denaturierenden Agarose-Gelelektrophorese und ompA Northern Blot Analyse von *E. coli* RNA isoliert mit Lösungen verschiedener Volumina und Konzentrationen wieder.

### Beispiel 4

### Stabilisierung von E. coli RNA

Zur Beurteilung der Stabilisierungseffizienz werden in diesem Beispiel Experimente durchgeführt, in denen den *E. coli* Zellen im Kulturmedium der RNA-Polymerase-Inhibitor Rifampicin zugegeben wird (FEBS Letters 1998, 440: 172 - 174). Damit wird die Neusynthese von RNA-Transkripten verhindert, wodurch die Analyse der Degradierung von RNA-Transkripten erleichtert wird. Zu definierten Zeitpunkten nach Zugabe des Inhibitors erfolgt die RNA-Isolierung aus den Zellen. Als Kontrollen dienen Ansätze, mit denen analog verfahren wird, jedoch die RNA ohne Zusatz der Lösung isoliert wird (RNeasy Standard-Protokoll). Zur Beurteilung der Intaktheit der mRNA werden nach der RNA-Isolierung Northern Blot Experimente durchgeführt.

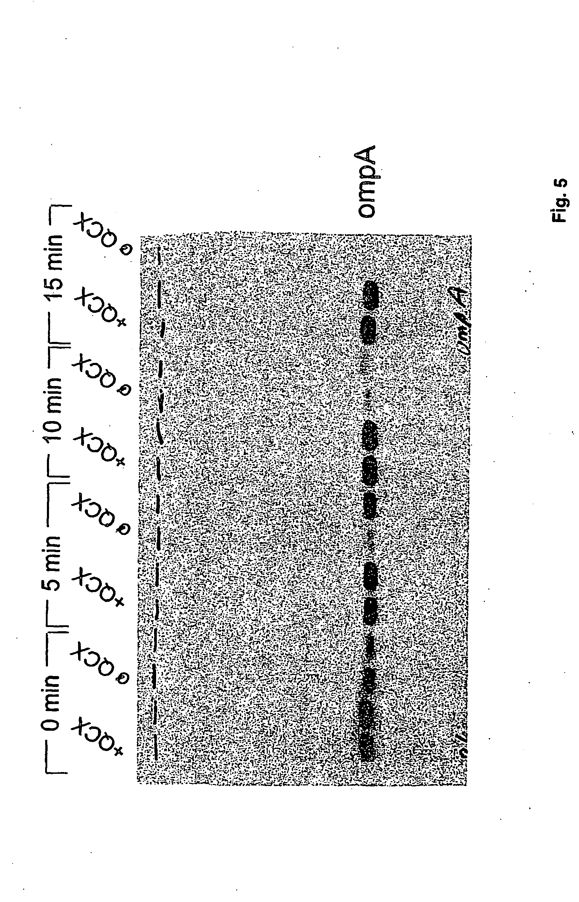

Fig. 5 zeigt die ompA ("outer membrane protein A") Northern Blot Analyse von *E*. *coli* RNA isoliert nach Rifampicin-Zugabe mit bzw. ohne Lösung der erfindungsgemäßen Komposition.

Bei der ompA mRNA handelt es sich um ein *E*. *coli* Transkript, daß unter den gewählten Kulturbedingungen eine Halbwertszeit von 15 Minuten aufweist (Nature 1984, 312: 75 - 77). Die Northern Blot Analyse (Fig. 5) zeigt, daß in den Proben, die mit der erfindungsgemäßen Lösung behandelt werden, über den gesamten untersuchten Zeitraum (bis zu 15 Minuten) ein gleichmäßig intensives Signal für die ompA mRNA zu detektieren ist. Im Gegensatz dazu ist für die ompA mRNA in den Proben ohne Zusatz des Detergenz bereits nach 0 bis 5 Minuten ein deutlicher Verlust des Transkripts zu verzeichnen.

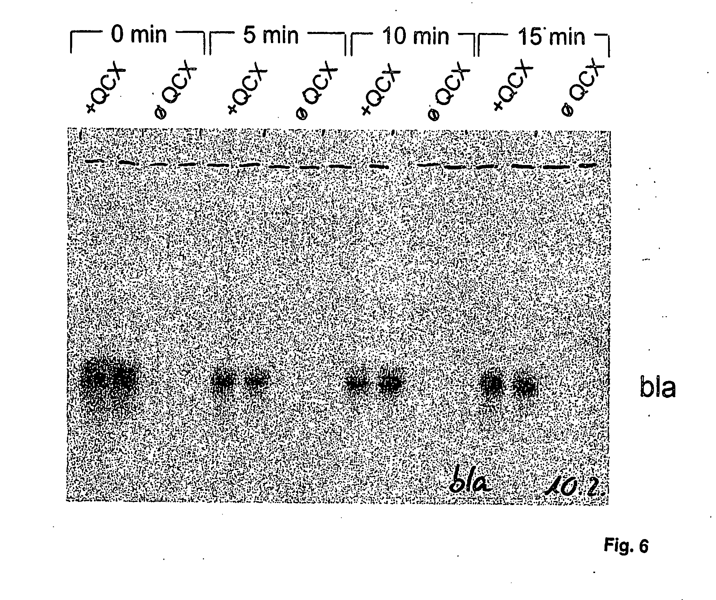

Fig. 6 zeigt die bla (β-lactamase) Northern Blot Analyse von *E. coli* RNA isoliert nach Rifampicin-Zugabe mit bzw. ohne Verwendung der Komposition bzw. der wässeriger Lösung aus katiionischer Verbindung und Additiv.

Bei einer Northern Blot Analyse für die β-lactamase mRNA (bla) ist derselbe Effekt noch deutlicher nachzuweisen. Dieses mRNA Transkript besitzt unter den gewählten Kulturbedingungen eine Halbwertszeit von 2-5 Minuten (Nature 1984, 312: 75-77). Wie aus Fig. 6 ersichtlich ist, ist dieses Transkript mit vergleichbarer Signalintensität über den gesamten untersuchten Zeitraum in den RNA-Proben nachzuweisen, die unter Zusatz der Lösung isoliert werden. In den Kontrollansätzen ohne die Detergenz-Lösung ist dagegen bereits bei sofortiger Aufarbeitung der RNA (0 Minuten) ein fast vollständiger Verlust des bla mRNA Transkripts zu verzeichnen. Die Differenz der bla mRNA Signalintensitäten zwischen den beiden RNA-Isolierungsmethoden reflektiert die unmittelbare Stabilisierung der mRNA durch die entsprechende wässerige Lösung der erfindungsgemäßen Komposition.

Diese Experimente belegen, daß mit der erfindungsgemäßen Komposition die Möglichkeit eröffnet wird, RNA-Expressionsprofile von Bakterien im Zustand der Flüssigkultur zu fixieren, ohne daß dabei Artefakte aus der Isolierungsprozedur zu einer Verzerrung des Expressionsmusters führen.

### Beispiel 5

### RNA-Stabilisierung mit Tetradecyltrimethylammoniumbromid (TTAB)

In den folgenden Experimenten wird eine TTAB-Lösung folgender Zusammensetzung verwendet:
4% (w/v) TTAB
200mM Weinsäure
pH 4,0

Verschiedene Bakterien-Species unterscheiden sich u.a. in der Beschaffenheit ihrer Zellwand. Beim Einsatz unterschiedlicher Species ist der Zellaufschluß ein kritischer Schritt zur Isolierung von RNA. Gegenüber dem enzymatischen Zellaufschluß, der insbesondere für Gram-negative Bakterienspezies zu guten Ergebnissen führt, bietet ein mechanischer Zellaufschluß potentiell die Möglichkeit, verschiedenste Species aufzuschließen.

Die nachfolgende Tablelle zeigt einen Vergleich der verschiedenen Ausbeuten, die zum einen nach Methoden des Standes der Technik (RNeasy unter Durchführung eines Lysozym-vermittelten Zellaufschlußes), unter Verwendung der erfindungsgemäßen Komposition und RNeasy inklusive des Lysozym-Verdaus, sowie unter Verwendung der erfindungsgemäßen Komposition und RNeasy, wobei der enzymatische Zellaufschluß durch den Einsatz der Kugelmühle unterstützt wurde. Bei der Verwendung der Kugelmühle (MM 300 der Firma QIAGEN) wurden pro Ansatz 50 mg Säure-gewaschene Glasbeads (Durchmesser 150-600 µm) verwendet. Der Zellaufschluß in der Kugelmühle erfolgte für 5 min bei maximaler Schüttelgeschwindigkeit (30 Hz).

**Tab. 2**

| RNA-Ausbeuten aus 1 x 10⁸ Zellen *B. subtilis* bei verschiedenen Zellaufschlußmethoden und anschließender RNA-Isolierung mit RNeasy® | | | |
|---|---|---|---|
| Zellaufschluß | Lysozymverdau | TTAB-Lösung + Lysozymverdau | TTAB-Lösung + Kugelmühle + Lysozymverdau |
| *B. subtilis* in LB-Medium | 7 µg | 15 µg | 19 µg |
| *B. subtilis* in Mineral-Medium | 3 µg | 8 µg | 10 µg |

Wie aus dem Vergleich deutlich ersichtlich ist, führt die RNA-Isolierung mit der Lösung der erfindungsgemäßen Komposition in Kombination mit einem mechanischen Zellaufschluß zu einer ca. 25 %-igen Erhöhung der Steigerung im Vergleich zum enzymatischen Zellaufschluß unter Verwendung der Lösung (Tab. 2). Im Vergleich zur RNA-Isolierung nach RNeasy Standardprotokoll wurde durch den mechanischen Zellaufschluß unter Einsatz der Detergenz-Lösung im Schnitt eine Verdreifachung der Ausbeute erzielt.

Diese Ergebnisse offenbaren einen deutlich Hinweis, daß der enzymatische Zellaufschluß unter Einsatz der Detergenz-Lösung auch bei den Gram-positiven *B*. *subtilis* Zellen effizient verläuft, so daß hier optional auf einen mechanischen Zellaufschluß verzichtet werden kann.

Die Verwendung einer mechanischen Aufschlußmethode - wie der Kugelmühle - eröffnet aber zudem die Möglichkeit, unter Verzicht auf einen enzymatischen Zellaufschluß eine ca. 6-fache Ausbeutesteigerung im Vergleich zur RNA-Isolierung ohne enzymatischen oder mechanischen Zellaufschluß zu erreichen, wie der nachfolgende experimentelle Befund eindeutig belegt:

Als Ausgangsmaterial dient eine in LB-Medium gewachsene Kultur von Bacillus subtilis. Verglichen werden die RNA-Ausbeuten aus jeweils 1,8 × 10⁸ Zellen pro Ansatz (Tab. 3). In einem Teil der Proben erfolgte der Zellaufschluß mit Hilfe einer Kugelmühle (MM300 der Firma QIAGEN) unter Einsatz von 50mg Säuregewaschenen Glasbeads (Durchmesser 150-600 µm) pro Ansatz verwendet. Der Zellaufschluß in der Kugelmühle erfolgte für 5 min bei maximaler ,

Schüttelgeschwindigkeit (30 Hz). In einem anderen Teil der Proben wurde weder ein enzymatischer noch ein meachanischer Zellaufschluß durchgeführt (Literaturstelle: J. Microbiol. Methods 44 (2001): 235 - 238) Promega "SV Total RNA Isolation System"

**Tab. 3**

| RNA-Ausbeute aus Bacillus subtilis in Abhängigkeit von der Zell-Aufschlußmethode und der Zugabe der TTAB-Lösung | | |
|---|---|---|
| | Ohne enzymatischen oder mechanischen Zellaufschluß | Mechanischer Zellaufschluß |
| Verwendung der TTAB-Lösung | 3µg | 18 µg |

## Patentansprüche

1. Verwendung einer Komposition umfassend als Bestandteile eine kationische Verbindung der allgemeinen Formel
Y⁺R₁ R₂R₃R₄ X⁻
worin
Y Stickstoff oder Phosphor
R₁, R₂, R₃ und R₄ unabhängig voneinander einen unverzweigten oder verzweigten C₁-C₂₀-Alkylrest und/oder einen C₆-C₂₀-Arylrest sowie einen C₇-C₂₆-Aralkylrest und
X⁻ ein Anion einer anorganischen oder organischen, ein- oder mehrbasischen Säure
bedeuten können
und mindestens einen Protonendonor
zur Stabilisierung und/oder Isolierung von Nukleinsäuren in bzw. aus Mikroorganismen.

2. Verwendung einer Komposition nach Anspruch 1, **dadurch gekennzeichnet, dass** die Mikroorganismen ausgewählt werden aus Prokaryonten, Pilzen, Protozoen oder Algen.

3. Verwendung einer Komposition nach Anspruch 1 oder 2, **dadurch gekennzeichnet, dass** Y Stickstoff bedeutet.

4. Verwendung einer Komposition nach einem der Ansprüche 1 bis 3, **dadurch gekennzeichnet, dass** R₁ einen C₇-C₂₀-Alkylrest und R₂, R₃ und R₄ jeweils eine Methylgruppe bedeutet.

5. Verwendung einer Komposition nach Anspruch 4, **dadurch gekennzeichnet, dass** R₁ einen Alkylrest mit 12, 14 oder 16 Kohlenstoffatomen und R₂, R₃ und R₄ jeweils eine Methylgruppe bedeutet.

6. Verwendung einer Komposition nach einem der Ansprüche 1 bis 5, **dadurch gekennzeichnet, dass** das Anion X⁻ aus der Gruppe der Anionen von Halogenwasserstoffsäuren oder Anionen ein- oder zweibasischer organischer Säuren ausgewählt wird.

7. Verwendung einer Komposition nach Anspruch 6, **dadurch gekennzeichnet, dass** das Anion X⁻ Anionen aus der Gruppe Bromid, Chlorid, Phosphat, Sulfat, Formiat, Acetat, Propionat, Oxalat, Malonat, Succinat oder Citrat ausgewählt wird.

8. Verwendung einer Komposition nach einem der Ansprüche 1 bis 7 **dadurch gekennzeichnet, dass** der Protonendonor aus der Gruppe der gesättigten aliphatischen Monocarbonsäuren, der ungesättigten Alkenyl-carbonsäuren, der gesättigten und/oder ungesättigten aliphatische C₂-C₆-Dicarbonsäuren und/oder Tricarbonsäuren, der aliphatischen Ketodicarbonsäuren, der Aminosäuren oder aus der Gruppe der Mineralsäuren oder deren Salze allein oder in Kombination ausgewählt wird.

9. Verwendung einer Komposition nach Anspruch 8, **dadurch gekennzeichnet, dass** als aliphatische Monocarbonsäure eine C₁-C₆-Alkyl-carbonsäure, vorzugsweise Essigsäure, Propionsäure, n-Buttersäure, n-Valeriansäure, Isovaleriansäure, Ethyl-methyl-essigsäure (2-Methyl-buttersäure), 2,2-Dimethylpropionsäure (Pivalinsäure), n-Hexansäure, n-Octansäure, n-Decansäure bzw. n-Dodecansäure (Laurinsäure) oder Mischungen der genannten Säuren eingesetzt werden.

10. Verwendung einer Komposition nach Anspruch 8, **dadurch gekennzeichnet, dass** als aliphatische Alkenyl-carbonsäure Acrylsäure (Propensäure), Methacrylsäure, Crotonsäure, iso-Crotonsäure oder Vinylessigsäure oder Mischungen der genannten Säuren eingesetzt werden.

11. Verwendung einer Komposition nach Anspruch 8, **dadurch gekennzeichnet** als gesättigte aliphatische C₂-C₆-Dicarbonsäure eine Dicarbonsäure aus der Gruppe Oxalsäure, Malonsäure, Bersteinsäure, Glutarsäure bzw. Adipinsäure oder Mischungen der genannten Säuren eingesetzt werden.

12. Verwendung einer Komposition nach Anspruch 11, **dadurch gekennzeichnet, dass** als Protonenedonoren aliphatische Dicarbonsäuren, vorzugsweise Oxalsäure oder Bersteinsäure oder Mischungen der genannten Säuren eingesetzt werden.

13. Verwendung einer Komposition nach Anspruch 8, **dadurch gekennzeichnet, dass** als Protonendonoren aliphatische Hydroxi-di- und -tricarbonsäuren, vorzugsweise Tartronsäure, D-(+)-, L-(-)- oder DL-Äpfelsäure, (2R, 3R)-(+)-Weinsäure, (2S, 3S)-(-)-Weinsäure, meso-Weinsäure und Citronensäure oder Mischungen der genannten Säuren eingesetzt werden.

14. Verwendung einer Komposition nach Anspruch 8, **dadurch gekennzeichnet, dass** als ungesättigte Dicarbonsäuren Malein- und/oder Fumarsäure oder Mischungen der genannten Säuren eingesetzt werden.

15. Verwendung einer Komposition nach Anspruch 6, **dadurch gekennzeichnet, dass** als ungesättigte Tricarbonsäuren Aconitsäure eingesetzt wird.

16. Verwendung einer Komposition nach Anspruch 8, **dadurch gekennzeichnet, dass** als aliphatische Ketodicarbonsäuren Mesoxalsäure oder Oxalessigsäure oder Mischungen der genannten Säuren eingesetzt werden.

17. Verwendung einer Komposition nach Anspruch 8, **dadurch gekennzeichnet, dass** als Aminosäuren Aminoessigsäure (Glycin), α-Aminopropionsäure (Alanin), α-Amino-*iso*-valeriansäure (Valin), α-Amino-*iso*-capronsäure (Leucin) und α-Amino-β-methylvaleriansäure (Isoleucin), oder Mischungen der genannten Säuren eingesetzt werden.

18. Verwendung einer Komposition nach einem der Ansprüche 1 bis 17, **dadurch gekennzeichnet, dass** die Komposition in Form einer wässerigen Lösung eingesetzt wird.

19. Verwendung einer Komposition nach Anspruch 18, **dadurch gekennzeichnet, dass** die kationische Verbindung in der Komposition in einer Konzentration in einem Intervall von 0.01 % (W/V) bis zur Sättigung vorliegt.

20. Verwendung einer Komposition nach Anspruch 19, **dadurch gekennzeichnet, dass** die kationische Verbindung in der Komposition in einer Konzentration zwischen 0.1 und 10 % (W/V) vorliegt.

21. Verwendung einer Komposition nach Anspruch 20, **dadurch gekennzeichnet, dass** die kationische Verbindung in der Komposition in einer Konzentration zwischen 0.5 und 8 % (W/V) vorliegt.

22. Verwendung einer Komposition nach Anspruch 21, **dadurch gekennzeichnet, dass** die kationische Verbindung in der Komposition in einer Konzentration zwischen 2 und 6 % (W/V) vorliegt.

23. Verwendung einer Komposition gemäß einem der Ansprüche 1 bis 22 **dadurch gekennzeichnet, dass** die Nukleinsäuren aus Prokaryonten, den Archaebakterien oder den Eubakterien stammen.

24. Verwendung nach Anspruch 23, **dadurch gekennzeichnet, dass** die Nukleinsäuren aus Gram-positiven Bakterien stammen.

25. Verwendung nach Anspruch 24, **dadurch gekennzeichnet, dass** die Nukleinsäuren aus Bacillus, Staphylococcus, Streptomyces, Falvobakterium, Mycobakterium, Streptococcus, Clostridien, Lysteria, Peptococcus, Peptostreptococcus, Enterococcus, Corynebacterium, Propionibakterium oder Lactobacillus stammen.

26. Verwendung nach Anspruch 23, **dadurch gekennzeichnet, dass** die Nukleinsäuren aus Gram-negativen Bakterien stammen.

27. Verwendung nach Anspruch 26, **dadurch gekennzeichnet, dass** die Nukleinsäuren aus Escherichia, Pseudomonas, Klebsiella, Salmonella, Sinorhizobium, Campylobacter, Neisseria, Vibrio, Shigella, Serratia, Enterobacter, Acinetobacter, Proteus, Yersinia, Brucella, Haemophilus, Bacteroides, Helicobacter, Bordetella, Legionella oder Pasteurella stammen.

28. Verwendung nach Anspruch 23, **dadurch gekennzeichnet, dass** die Nukleinsäuren aus Clamydien stammen.

29. Verwendung nach Anspruch 23, **dadurch gekennzeichnet, dass** die Nukleinsäuren aus phototrophen Bakterien stammen.

30. Verwendung nach Anspruch 23, **dadurch gekennzeichnet, dass** die Nukleinsäuren aus Mycoplasma - wie z.B. Mycoplasma penetrans - stammen.

31. Verwendung nach Anspruch 30, **dadurch gekennzeichnet, dass** die Nukleinsäuren aus Mycoplasma penetrans stammen.

32. Verwendung nach Anspruch 23, **dadurch gekennzeichnet, dass** die Nukleinsäuren aus Rikettsien stammen.

33. Verwendung nach Anspruch 23, **dadurch gekennzeichnet, dass** die Nukleinsäuren aus Spirochaeten stammen.

34. Verwendung nach Anspruch 23, **dadurch gekennzeichnet, dass** die Nukleinsäuren aus Spirillen stammen.

35. Verwendung nach einem der Ansprüche 1 bis 22, **dadurch gekennzeichnet, dass** die Nukleinsäuren aus eukaryotischen Mikroorganismen stammen.

36. Verwendung nach Anspruch 35, **dadurch gekennzeichnet, dass** die Nukleinsäuren aus Pilzen der Gruppe der Dermatophyten, der Hefen, der Schimmelpilze und der biphasischen Pilze stammen.

37. Verwendung nach Anspruch 36, **dadurch gekennzeichnet, dass** die Nukleinsäuren aus den Hefen Saccaromyces, Candida oder Cryptococcus stammen.

38. Verwendung nach Anspruch 36 , **dadurch gekennzeichnet, dass** die Nukleinsäuren aus Schimmelpilzen der Gruppe Aspergillus stammen.

39. Verwendung nach Anspruch 36, **dadurch gekennzeichnet, dass** die Nukleinsäuren aus Schimmelpilzen der Gruppe Penicillium stammen.

40. Verwendung nach Anspruch 36 , **dadurch gekennzeichnet, dass** die Nukleinsäuren aus Schimmelpilzen der Gruppe Mucor stammen.

41. Verwendung nach Anspruch 35, **dadurch gekennzeichnet, dass** die Nukleinsäuren aus Algen stammen.

42. Verwendung nach Anspruch 35, **dadurch gekennzeichnet, dass** die Nukleinsäuren aus Protozoen stammen.

43. Verwendung nach Anspruch 42, **dadurch gekennzeichnet, dass** die Protozoen ausgewählt sind aus Trypanosomen, Toxoplasmen, Amoeben, Plasmodien oder Flagellaten.

44. Verwendung einer Komposition gemäß einem der Ansprüche 1 bis 43, **dadurch gekennzeichnet, dass** ein enzymatischer, mechanischer, thermischer oder chemischer Aufschluß der Bakterien oder der Pilze oder der Protozoen oder der Algen erfolgt oder eine Kombination der Aufschlußmethoden angewendet wird.

45. Verwendung einer Komposition gemäß einem der Ansprüche 1 bis 44, **dadurch gekennzeichnet, dass** der pH-Wert der Komposition in einem Bereich von 2 bis 12 liegt.

46. Verwendung einer Komposition nach Anspruch 45, **dadurch gekennzeichnet, dass** der pH-Wert der Komposition in einem Bereich von 2 bis 8 liegt.

47. Verwendung einer Komposition nach Anspruch 46, **dadurch gekennzeichnet, dass** der pH-Wert der Komposition in einem Intervall von 2 bis 5 liegt.

## Claims

1. Use of a composition comprising as components a cationic compound of the general formula
Y⁺R₁R₂R₃R₄ X⁻
wherein
Y can mean nitrogen or phosphorus,
R₁, R₂, R₃ and R₄ independently of one another can mean a linear or branched C₁-C₂₀ alkyl residue and/or a C₆-C₂₀ aryl residue as well as a C₇-C₂₆ aralkyl residue and
X⁻ can mean an anion of an inorganic or organic mono- or polyprotic acid
and at least one proton donor
for the stabilisation and/or isolation of nucleic acids in or respectively from microorganisms.

2. Use of a composition according to claim 1, **characterised in that** the microorganisms are selected from prokaryotes, fungi, protozoa or algae.

3. Use of a composition according to claim 1 or 2, **characterised in that** Y means nitrogen.

4. Use of a composition according to any of the claims 1 to 3, **characterised in that** R₁ means a C₇-C₂₀ alkyl residue and R₂, R₃ and R₄ mean in each case a methyl group.

5. Use of a composition according to claim 4, **characterised in that** R₁ means an alkyl residue with 12, 14 or 16 carbon atoms and R₂, R₃ and R₄ mean in each case a methyl group.

6. Use of a composition according to any of the claims 1 to 5, **characterised in that** the anion X⁻ is selected from the group of anions of hydrohalic acids or anions of mono- or diprotic organic acids.

7. Use of a composition according to claim 6, **characterised in that** the anion X⁻ is selected from anions from the group bromide, chloride, phosphate, sulphate, formate, acetate, propionate, oxalate, malonate, succinate or citrate.

8. Use of a composition according to any of the claims 1 to 7, **characterised in that** the proton donor is selected from the group of the saturated aliphatic monocarboxylic acids, the unsaturated alkenylcarboxylic acids, the saturated and/or unsaturated aliphatic C₂-C₆ dicarboxylic acids and/or tricarboxylic acids, the aliphatic ketodicarboxylic acids, the amino acids or from of the group of the mineral acids or their salts, alone or in combination.

9. Use of a composition according to claim 8, **characterised in that** as aliphatic monocarboxylic acid a C₁-C₆ alkylcarboxylic acid, preferably acetic acid, propionic acid, n-butyric acid, n-valeric acid, isovaleric acid, ethylmethylacetic acid (2-methylbutyric acid), 2,2-dimethylpropionic acid (pivalic acid), n-hexanoic acid, n-octanoic acid, n-decanoic acid or n-dodecanoic acid (lauric acid) or mixtures of said acids are used.

10. Use of a composition according to claim 8, **characterised in that** as aliphatic alkenylcarboxylic acid acrylic acid (propenoic acid), methacrylic acid, crotonic acid, iso-crotonic acid or vinylacetic acid or mixtures of said acids are used.

11. Use of a composition according to claim 8, **characterised in that** as saturated aliphatic C₂-C₆ dicarboxylic acid a dicarboxylic acid from the group of oxalic acid, malonic acid, succinic acid, glutaric acid or adipic acid or mixtures of said acids are used.

12. Use of a composition according to claim 11, **characterised in that** as proton donors aliphatic dicarboxylic acids, preferably oxalic acid or succinic acid or mixtures of said acids are used.

13. Use of a composition according to claim 8, **characterised in that** as proton donors aliphatic hydroxy di- and -tricarboxylic acids, preferably tartronic acid (hydroxymalonic acid), D-(+)-, L-(-)- or DL-malic acid, (2R, 3R)-(+)-tartaric acid, (2S, 3S)-(-)-tartaric acid, meso-tartaric acid and citric acid or mixtures of said acids are used.

14. Use of a composition according to claim 8, **characterised in that** as unsaturated dicarboxylic acids maleic and/or fumaric acid or mixtures of said acids are used.

15. Use of a composition according to claim 8, **characterised in that** as unsaturated tricarboxylic acid aconitic acid is used.

16. Use of a composition according to claim 8, **characterised in that** as aliphatic ketodicarboxylic acid mesoxalic acid or oxalacetic acid or mixtures of said acids are used.

17. Use of a composition according to claim 8, **characterised in that** as amino acids aminoacetic acid (glycine), α-aminopropionic acid (alanine), α-amino-*iso*-valeric acid (valine), α-amino-*iso*-caproic acid (leucine) and α-amino-β-methylvaleric acid (isoleucine), or mixtures of said acids are used.

18. Use of a composition according to any of the claims 1 to 17, **characterised in that** the composition is used in the form of an aqueous solution.

19. Use of a composition according to claim 18, **characterised in that** the cationic compound is present in the composition in a concentration in a range of 0.01 % (w/v) up to saturation.

20. Use of a composition according to claim 19, **characterised in that** the cationic compound is present in the composition in a concentration between 0.1 and 10 % (w/v).

21. Use of a composition according to claim 20, **characterised in that** the cationic compound is present in the composition in a concentration between 0.5 and 8 % (w/v).

22. Use of a composition according to claim 21, **characterised in that** the cationic compound is present in the composition in a concentration between 2 and 6 % (w/v).

23. Use of a composition according to any of the claims 1 bis 22, **characterised in that** the nucleic acids originate from prokaryotes, the archaebacteria or the eubacteria.

24. Use according to claim 23, **characterised in that** the nucleic acids originate from gram-positive bacteria.

25. Use according to claim 24, **characterised in that** the nucleic acids originate from Bacillus, Staphylococcus, Streptomyces, Falvobacterium, Mycobacterium, Streptococcus, Clostridia, Lysteria, Peptococcus, Peptostreptococcus, Enterococcus, Corynebacterium, Propionibacterium or Lactobacillus.

26. Use according to claim 23, **characterised in that** the nucleic acids originate from gram-negative bacteria.

27. Use according to claim 26, **characterised in that** the nucleic acids originate from Escherichia, Pseudomonas, Klebsiella, Salmonella, Sinorhizobium, Campylobacter, Neisseria, Vibrio, Shigella, Serratia, Enterobacter, Acinetobacter, Proteus, Yersinia, Brucella, Haemophilus, Bacteroides, Helicobacter, Bordetella, Legionella or Pasteurella.

28. Use according to claim 23, **characterised in that** the nucleic acids originate from Clamydia.

29. Use according to claim 23, **characterised in that** the nucleic acids originate from phototrophic bacteria.

30. Use according to claim 23, **characterised in that** the nucleic acids originate from Mycoplasma.

31. Use according to claim 30, **characterised in that** the nucleic acids originate from Mycoplasma penetrans.

32. Use according to claim 23, **characterised in that** the nucleic acids originate from Rickettsia.

33. Use according to claim 23, **characterised in that** the nucleic acids originate from Spirochaeta.

34. Use according to claim 23, **characterised in that** the nucleic acids originate from Spirilla.

35. Use according to any of the claims 1 to 22, **characterised in that** the nucleic acids originate from eukaryotic microorganisms.

36. Use according to claim 35, **characterised in that** the nucleic acids originate from fungi of the group of the Dermatophytes, yeasts, moulds and the biphasic fungi.

37. Use according to claim 36, **characterised in that** the nucleic acids originate from the yeast Saccaromyces, Candida or Cryptococcus.

38. Use according to claim 36, **characterised in that** the nucleic acids originate from moulds of the group Aspergillus.

39. Use according to claim 36, **characterised in that** the nucleic acids originate from moulds of the group Penicillium.

40. Use according to claim 36, **characterised in that** the nucleic acids originate from moulds of the group Mucor.

41. Use according to claim 35, **characterised in that** the nucleic acids originate from algae.

42. Use according to claim 35, **characterised in that** the nucleic acids originate from protozoa.

43. Use according to claim 42, **characterised in that** the protozoa are selected from trypanosomes, toxoplasma, amoeba, plasmodia or flagellates.

44. Use of a composition according to any of the claims 1 to 43, **characterised in that** an enzymatic, mechanical, thermal or chemical digestion of the bacteria or the fungi or the protozoa or the algae is carried out or a combination of the digestion methods is used.

45. Use of a composition according to any of the claims 1 to 44, **characterised in that** the pH value of the composition lies in a range of 2 to 12.

46. Use of a composition according to claim 45, **characterised in that** the pH value of the composition lies in a range of 2 to 8.

47. Use of a composition according to claim 46, **characterised in that** the pH value of the composition lies in a range of 2 to 5.

## Revendications

1. Utilisation d'une composition comprenant en tant que constituant un composé cationique répondant à la formule générale suivante :
Y⁺R₁R₂R₃R₄ X⁻
dans laquelle
Y peut désigner un atome d'azote ou de phosphore
R₁, R₂, R₃ et R₄ sont indépendamment les uns des autres un résidu alkyle en C₁ à C₂₀ ramifié ou linéaire et/ou un résidu aryle en C₆ à C₂₀ ainsi qu'un résidu aralkyle en C₇ à C₂₆ et
X⁻ peut désigner un anion d'un acide polybasique ou monobasique organique ou inorganique,
et au moins un donneur de protons
en vue de stabiliser et/ou d'isoler des acides nucléiques dans ou à partir de micro-organismes.

2. Utilisation d'une composition selon la revendication 1, **caractérisée en ce que** les micro-organismes sont sélectionnés parmi des procaryotes, des champignons, des protozoaires ou des algues.

3. Utilisation d'une composition selon la revendication 1 ou 2, **caractérisée en ce que** Y désigne un atome d'azote.

4. Utilisation d'une composition selon l'une quelconque des revendications 1 à 3, **caractérisée en ce que** R₁ désigne un résidu alkyle en C₇ à C₂₀ et R₂, R₃ et R₄ désignent chacun un groupe méthyle.

5. Utilisation d'une composition selon la revendication 4, **caractérisée en ce que** R₁ désigne un résidu alkyle ayant 12, 14 ou 16 atomes de carbone et R₂, R₃ et R₄ désignent chacun un groupe méthyle.

6. Utilisation d'une composition selon l'une quelconque des revendications 1 à 5, **caractérisée en ce que** l'anion X⁻ est sélectionné dans le groupe des anions d'haloacides ou des anions d'acides organiques monobasiques ou bibasiques.

7. Utilisation d'une composition selon la revendication 6, **caractérisée en ce que** l'anion X⁻ est sélectionné parmi des anions du groupe bromure, chlorure, phosphate, sulfate, formiate, acétate, propionate, oxalate, malonate, succinate ou citrate.

8. Utilisation d'une composition selon l'une quelconque des revendications 1 à 7, **caractérisée en ce que** le donneur de protons est sélectionné dans le groupe des acides monocarboxyliques aliphatiques saturés, des acides carboxyliques d'alcényle insaturés, des acides tricarboxyliques et/ou dicarboxyliques en C₂ à C₆ aliphatiques insaturés et/ou saturés, des acides cétodicarboxyliques aliphatiques, des acides aminés ou dans le groupe des acides minéraux ou de leurs sels seuls ou en combinaison.

9. Utilisation d'une composition selon la revendication 8, **caractérisée en ce qu'**un acide carboxylique d'alkyle en C₁ à C₆, de préférence un acide acétique, un acide propionique, un acide n-butyrique, un acide n-valérique, un acide isovalérique, un acide éthyl-méthyl acétique, (un acide 2-méthylbutyrique), un acide 2,2-diméthylpropionique (acide pivalique), un acide n-hexanoïque, un acide n-octanoïque, un acide n-décanoïque ou un acide n-dodécanoïque, (acide laurique) ou des mélanges des acides cités sont utilisés en tant qu'acide monocarboxylique aliphatique.

10. Utilisation d'une composition selon la revendication 8, **caractérisée en ce que** de l'acide acrylique (acide propénique), de l'acide méthacrylique, de l'acide crotonique, de l'acide iso-crotonique ou de l'acide acétique de vinyle ou des mélanges des acides cités sont utilisés en tant qu'acide carboxylique d'alcényle aliphatique.

11. Utilisation d'une composition selon la revendication 8, **caractérisée en ce qu'**un acide dicarboxylique provenant du groupe de l'acide oxalique, de l'acide malonique, de l'acide Berstein, de l'acide glutarique ou de l'acide adipique ou des mélanges des acides cités sont utilisés en tant qu'acide dicarboxylique en C₂ à C₆ aliphatique saturé.

12. Utilisation d'une composition selon la revendication 11, **caractérisée en ce que** des acides dicarboxyliques aliphatiques, de préférence un acide oxalique ou un acide Berstein ou des mélanges des acides cités sont utilisés en tant que donneur de protons.

13. Utilisation d'une composition selon la revendication 8, **caractérisée en ce que** des acides hydroxytricarboxyliques et hydroxydicarboxyliques aliphatiques, de préférence de l'acide tartronique, de l'acide D -(+)-, L-(-)-malique ou de l'acide DL-malique, de l'acide (2R, 3R)-(+)-tartrique, de l'acide (2S, 3S)-(-)-tartrique, de l'acide mésotartrique et de l'acide citrique ou des mélanges des acides cités sont utilisés en tant que donneurs de protons.

14. Utilisation d'une composition selon la revendication 8, **caractérisée en ce que** des acides maléiques et/ou fumariques ou des mélanges des acides cités sont utilisés en tant qu'acide dicarboxylique insaturé.

15. Utilisation d'une composition selon la revendication 8, **caractérisée en ce qu'**un acide aconitique est utilisé en tant qu'acide tricarboxylique insaturé.

16. Utilisation d'une composition selon la revendication 8, **caractérisée en ce que** de l'acide mésoxalique ou de l'acide oxalo-acétique ou des mélanges des acides cités sont utilisés en tant qu'acides cétodicarboxyliques aliphatiques.

17. Utilisation d'une composition selon la revendication 8, **caractérisée en ce que** de l'acide aminoacétique (glycine), de l'acide α-aminopropionique (alanine), de l'acide α-amino-*iso*-valérique (valine), de l'acide α-amino-*iso*-capronique (leucine) et des acides α-amino-β-méthylvalérique (isoleucine) ou des mélanges des acides cités sont utilisés en tant qu'acides aminés.

18. Utilisation d'une composition selon l'une quelconque des revendications 1 à 17, **caractérisée en ce que** la composition est utilisée sous la forme d'une solution aqueuse.

19. Utilisation d'une composition selon la revendication 18, **caractérisée en ce que** le composé cationique est présent dans la composition à une concentration dans un intervalle allant de 0,01 % (P/V) à la saturation.

20. Utilisation d'une composition selon la revendication 19, **caractérisée en ce que** le composé cationique est présent dans la composition à une concentration entre 0,1 et 10 % (P/V).

21. Utilisation d'une composition selon la revendication 20, **caractérisée en ce que** le composé cationique est présent dans la composition à une concentration entre 0,5 et 8 % (P/V).

22. Utilisation d'une composition selon la revendication 21, **caractérisée en ce que** le composé cationique est présent dans la composition à une concentration entre 2 et 6 % (P/V).

23. Utilisation d'une composition selon l'une quelconque des revendications 1 à 22, **caractérisée en ce que** les acides nucléiques proviennent des procaryotes, des archaebactéries ou des eubactéries.

24. Utilisation selon la revendication 23, **caractérisée en ce que** les acides nucléiques proviennent de bactéries à Gram-positif.

25. Utilisation selon la revendication 24, **caractérisée en ce que** les acides nucléiques proviennent de bacilles, staphylocoques, streptomycètes, flavobacterium, Mycobacterium, Streptococcus, Clostridia, Lysteria, Peptococcus, Peptostreptococcus, Enterococcus, Corynebacterium, Propionibacterium ou Lactobacillus.

26. Utilisation selon la revendication 23, **caractérisée en ce que** les acides nucléiques proviennent de bactéries à Gram-négatif.

27. Utilisation selon la revendication 26, **caractérisée en ce que** les acides nucléiques proviennent de Escherichia, Pseudomonas, Klebsiella, Salmonella, Sinorhizobium, Campylobacter, Neisseria, Vibrio, Shigella, Serratia, Enterobacter, Acinetobacter, Proteus, Yersinia, Brucella, Haemophilus, Bacteroides, Helicobacter, Bordetella, Legionella ou Pasteurella.

28. Utilisation selon la revendication 23, **caractérisée en ce que** les acides nucléiques proviennent des Chlamydiae.

29. Utilisation selon la revendication 23, **caractérisée en ce que** les acides nucléiques proviennent des bactéries phototrophes.

30. Utilisation selon la revendication 23, **caractérisée en ce que** les acides nucléiques proviennent de mycoplasmes.

31. Utilisation selon la revendication 30, **caractérisée en ce que** les acides nucléiques proviennent de Mycoplasma penetrans.

32. Utilisation selon la revendication 23, **caractérisée en ce que** les acides nucléiques proviennent de Rickettsies.

33. Utilisation de la revendication 23, **caractérisée en ce que** les acides nucléiques proviennent de Spirochètes.

34. Utilisation selon la revendication 23, **caractérisée en ce que** les acides nucléiques proviennent de Spirilles.

35. Utilisation selon l'une quelconque des revendications 1 à 22, **caractérisée en ce que** les acides nucléiques proviennent de micro-organismes eucaryotes.

36. Utilisation selon la revendication 35, **caractérisée en ce que** les acides nucléiques proviennent de champignons du groupe des dermatophytes, des levures, des moisissures ou des champignons biphasiques.

37. Utilisation selon la revendication 36, **caractérisée en ce que** les acides nucléiques proviennent des levures Saccaromyces, Candida ou Cryptococcus.

38. Utilisation selon la revendication 36, **caractérisée en ce que** les acides nucléiques proviennent de moisissures du groupe Aspergillus.

39. Utilisation selon la revendication 36, **caractérisée en ce que** les acides nucléiques proviennent de moisissures du groupe Penicillium.

40. Utilisation selon la revendication 36, **caractérisée en ce que** les acides nucléiques proviennent de moisissures du groupe Mucor.

41. Utilisation selon la revendication 35, **caractérisée en ce que** les acides nucléiques proviennent d'algues.

42. Utilisation selon la revendication 35, **caractérisée en ce que** les acides nucléiques proviennent de protozoaires.

43. Utilisation selon la revendication 42, **caractérisée en ce que** les protozoaires sont sélectionnés parmi des trypanosomes, des toxoplasmes, des amibes, des plasmodes ou des flagelles.

44. Utilisation d'une composition selon l'une quelconque des revendications 1 à 43, **caractérisée en ce qu'**un traitement initial enzymatique, mécanique, thermique ou chimique des bactéries ou des champignons ou des protozoaires ou des algues est effectué ou qu'une combinaison des différentes méthodes de traitement est utilisée.

45. Utilisation d'une composition selon l'une quelconque des revendications 1 à 44, **caractérisée en ce que** la valeur de pH de la composition se trouve dans une plage allant de 2 à 12.

46. Utilisation d'une composition selon la revendication 45, **caractérisée en ce que** la valeur de pH de la composition s'inscrit dans une plage allant de 2 à 8.

47. Utilisation d'une composition selon la revendication 46, **caractérisée en ce que** la valeur de pH de la composition s'inscrit dans un intervalle allant de 2 à 5.
